# FASCICULE DE BREVET EUROPEEN

(11) **EP 0 374 061 B1**
(45) Date de publication et mention de la délivrance du brevet: **15.06.1994**
(21) Numéro de dépôt: 89420489.0
(22) Date de dépôt: 12.12.1989
(51) Int. Cl.: C07B 45/06, C07D 231/44

(54) **Procédé de préparation de perhalogénoalkylthioéthers**
Verfahren zur Herstellung von Perhalogenalkylthioethern
Process for the preparation of perhalogen alkyl thio ethers

(30) Priorité: 13.12.1988 FR 8816710; 09.10.1989 FR 8913371
(43) Date de publication de la demande: 20.06.1990
(73) Titulaire: RHONE-POULENC AGROCHIMIE, 69009 Lyon (FR)
(72) Inventeur: Clavel, Jean-Louis, F-69130 Ecully (FR); Langlois, Bernard, F-69006 Lyon (FR); Nantermet, Roland, F-69003 Lyon (FR); Tordeux, Marc, F-92330 Sceaux (FR); Wakselman, Claude, F-75016 Paris (FR)
(74) Mandataire: Brachotte, Charles

## Description

La présente invention a pour objet un procédé de préparation de perhalogénoalkylthioéthers ; elle concerne plus particulièrement la préparation de perhalogénoalkylthioéthers par réaction d'un disulfure avec un perhalogénoalcane en présence d'un réducteur.

On connait dans l'art antérieur divers procédés permettant l'obtention de perhalogénoalkythioéthers. On connait en particulier J.gen.Chem.USSR 1952, - 22 2273 et 1954,24,885 qui décrivent la préparation de divers trifluorométhylthiobenzènes par chloration du dérivé - SCH₃ correspondant en dérivé - SCCl₃ puis par fluoration de ce dernier en - SCF₃. Cette même méthode, permet, par fluoration incomplète, d'obtenir les dérivés - SCF₂Cl (voir Angew.Chem.Int.Ed,1977, 16,735).

On peut encore citer J.Org.Chem. 1964,29, 895 qui décrit la condensation du chlorure de trifluorométhane sulfényle CF₃SCl avec des organomagnésiens pour obtenir les dérivés - SCF₃.

Dans Synthesis 1975, 721 est décrit un procédé de préparation de dérivés - SCF₃, par réaction de CF₃SCu avec l'iodure correspondant.

Un autre procédé de l'art antérieur consiste à faire réagir CF₃I et un thiol sous irradiation ultraviolette dans de l'ammoniac liquide (J.Org.Chem.USSR 1977, 13, 972).

On citera enfin la préparation de dérivés SCF₂Br par action de CF₂Br₂ ou CF₂BrCl sur des thiophénates dans des conditions de transfert de phase liquide-liquide, l'agent de transfert de phase étant un sel d'ammonium quaternaire (Tetrahedron Letters 1981, 22, 323).

Ce dernier procédé ne permet pas la mise en oeuvre de CF₃Br et de CF₂Cl₂ et donc ne permet pas la préparation des dérivés - SCF₃ et SCF₂Cl.

Les autres procédés mentionnés présentent des inconvénients qui sont peu compatibles avec une exploitation industrielle et qui sont, principalement, le nombre élevé d'étapes nécessaires, la mise en oeuvre de produits chers et/ou toxiques comme CF₃I, CF₃SCl et CF₃SCu, le passage par un organomagnésien dont l'homme de l'art connaît bien les inconvénients ou encore la mise en oeuvre de réactions dans de l'ammoniac liquide.

Le brevet FR 2 540 108 concerne uniquement la préparation de phénylperfluoroalkylsulfures par action des thiophénates sur les halogénures de perfluoroalkyle. Cette méthode implique la formation préalable de thiophénates oxydables.

On doit également noter les brevets EP 201852 et 234119 (notamment EP 201852 pages 45 et 61- 62) qui décrivent la réaction d'halogénure d'alkyle avec le disulfure en présence de dithionite alcalin. Toutefois, cette méthode requiert la préparation préalable du thiolate et l'expérience a prouvé que cette méthode n'était pas valable dans le cas des halogénures de perfluoroalkyle.

Aucun des procédés décrits dans l'art antérieur n'est industrialisable. L'industrie cherche en effet un procédé peu onéreux, et surtout non dangereux, d'obtention des perhaloalkylsulfures demandant un nombre d'étapes de synthèse minimum.

La présente invention répond à cet objectif et a plus particulièrement pour objet un procédé de préparation de perhaloalkyllthioéthers par mise en contact, éventuellement dans un solvant,
- d'un réducteur formé d'un métal choisi parmi le zinc, le cadmium, l'aluminium, le manganèse, avec du dioxyde de soufre, ou formé d'un dithionite alcalin ou d'un hydroxyméthane sulfinate alcalin, alcalino terreux ou métallique ou formé d'un anion formiate et de dioxyde de soufre,
- d'un disulfure,
- d'un halogénure de perfluoroalkyle
Les perhalogénoalkylthioéthers répondent à la formule générale suivante :

R S C F Y T I

dans laquelle :
- R représente un groupe hydrocarbyle éventuellement substitué
- Y et T représentent indépendamment un halogène choisi parmi le fluor, le chlore et le brome ou une chaîne perhalogénoalkyle contenant 1 à 11 atomes de carbones.

Par chaîne perhalogénoalkyle on entend une chaîne dont tous les atomes d'hydrogène ont été remplacés par des atomes de chlore et/ou de brome et/ou de fluor et dans laquelle les atomes de chlore et/ou de brome ne sont pas vicinaux
Ils sont obtenus par réaction d'un disulfure et d'un halogénure de perfluoroalkyle selon la réaction suivante :

R - S- S- R+ 2 X C F T Y - - > 2 R - S - C F Y T

Dans ce procédé, les halogénures de perfluoroalkyle répondent à la formule :

X C F Y T II

dans laquelle
- X représente un halogène choisi parmi Cl, Br ou I,
- Y et T ont la même signification que dans la formule (I).

On peut citer comme composés de formule II le bromure de trifluorométhyle, l'iodure de perfluoroéthyle, le 1,1,2-trichloro trifluoroéthane, le trichlorofluorométhane, le trichloro-1,1,1 trifluoroéthane, le dibromodifluorométhane, dichlorodifluorométhane.

De préférence, quand X représente le chlore, Y ne peut représenter ni le fluor ni une chaîne perfluoroalkyle

De préférence,
- lorsque X représente le brome, Y et T représentent le fluor
- et lorsque X représente l'iode, Y représente un chaîne perfluoroalkyle, T un atome de fluor.

Par groupe hydrocarbyle on entend désigner notamment :
Un groupe carboacyclique (aliphatique) linéaire ou ramifié comportant une à cinq insaturations éthyléniques ou acétyléniques mais, de préférence, saturé.

Un groupe carbocyclique ou hétérocyclique choisi parmi un système monocyclique aromatique ou non aromatique (de préférence saturé), un système bicyclique aromatique ou non aromatique (de préférence saturé), un système polycyclique aromatique ou non aromatique (de préférence saturé), un système ponté (de préférence saturé).

Lorsque le terme polyhalogéno est utilisé dans le cas d'un substituant saturé, il signifie que les halogénes ne sont pas vicinaux, à l'exception du fluor.

Dans le cas des groupes carboacycliques (aliphatiques) les substituants (Z) admissibles sont identiques ou différents et sont choisis parmi les atomes d'halogène (I,Cl,Br,F); les radicaux C₆- C₁₀ aryle éventuellement substitués par 1 à 6 substituants choisis parmi les atomes d'halogène (I,Cl,Br,F), les radicaux C₁-C₆ alkyle, C₁- C₆ alkoxy, C₁- C₆ alkylthio, polyhalo C₁-C₆ alkyle, polyhalo C₁- C₆ alkoxy, polyhalo C₁- C₆ alkylthio, C₁- C₆ alkylsulfinyl, polyhalo C₁- C₆ alkylsulfinyl, cyano, C₁- C₆ alkyl sulfonyl ou polyhalo C₁- C₆ alkylsulfonyl, C₁- C₆ alkoxycarbonyl, polyhalo C₁- C₆ alkoxycarbonyl ; C₁- C₆ alkylcarbonyloxy ; polyhalo C₁- C₆ alkylcarbonyloxy ;un reste S(0)m- R₁ dans lequel R₁ est un groupe amino, C₁-C₆ alkyl amino,
di (C₁- C₆ alkyl) amino, polyhalo C₁- C₆ alkylamino,
di polyhalo C₁- C₆ alkylamino et m = 0,1,2 ; les radicaux C₁- C₉ hétéroaryle comportant en outre 1 à 4 hétéro atomes choisis parmi l'azote, le soufre, l'oxygène éventuellement substitué par l'un des substituants précédemment définis pour les radicaux C₆- C₁₀ aryle ; les radicaux C₁- C₆ alkoxy ; polyhalo C₁- C₆ alkoxy ; C₁-C₆ alkylthio ; polyhalo C₁- C₆ alkylthio ; C₁- C₆ alkylsufinyl ; polyhalo C₁- C₆ alkylsulfinyl; C₁- C₆ alkylsulfonyl ; polyhalo C₁- C₆ alkylsulfonyl ; C₁- C₆ alkoxycarbonyle ; polyhalo C₁- C₆ alkoxycarbonyl ; C₁-C₆ alkylcarbonyloxy ; polyhalo C₁- C₆ alkylcarbonyloxy ; hydroxy ; thiol ; un groupe amino NR₄R₅ dans lequel R₄, R₅ identiques ou différents représentent un atome d'hydrogène ; un groupe C₁- C₆ alkyle éventuellement substitué par un groupe C₂- C₅ alkoxycarbonyle ; C₃- C₆ cycloalkyle ; C₂- C₇ alcanoyle éventuellement formant ensemble un groupe imide cyclique de 5 à 6 atomes avec l'atome d'azote auquel ils sont attachés, lesdits groupes pouvant être éventuellement substitués par un à six atomes d'halogène ; C₂- C₇ alkoxycarbonyle ; polyhalo C₂-C₇ alkoxycarbonyle ; Z représente encore C₂- C₅ alkoxyméthylène éventuellement substitué sur le méthylène par un groupe C₁- C₄ alkyle ; carboxy tri C₁- C₆ alkylsilylmethyl ; tri C₁- C₆ alkylsilyl ; cyano ; Z représente encore un reste HN-C(=A)-R6,
- R6 étant un atome d'hydrogène, un radical C₁- C₆ alkyle ; C₂- C₄ alkenyle ; C₂- C₄ alkinyle ; C₁-C₄ alkoxyalkyle, C₁- C₄ alkylthioalkyle, C₁- C₄ alkoxy ; C₁- C₄ alkylthio, C₁- C₄ alkylamino, di(C₁- C₄ alkyl)amino ; polyhalo C₁- C₄ alkyle ; C₃- C₇ cycloalkyl éventuellement substitué par un ou plusieurs atomes d'halogène, C₁- C₄ alkyle ; C₁- C₄ halogènoalkyl ou R₆ représente encore un noyau phényl ; phénylthio ; phénoxy; phénylamino, ces noyaux phényl étant éventuellement substitués par les radicaux cyano, C₁- C₄ alkyl, C₁- C₄ alkoxy ; C₁- C₄ alkylthio ; C₁- C₄ alkylsulfinyle ; C₁-C₄ alkylsulfonyle ; polyhalo C₁- C₄ alkyle ; polyhalo C₁- C₄ alkoxy; polyhalo C₁- C₄ alkylthio ; polyhalo C₁-C₄ alkylsulfinyle ;polyhalo C₁- C₄ alkylsulfonyle; Z peut encore représenter un radical C₃- C₇ cycloalkyle, polyhalo C₃- C₇ cycloalkyle ou un radical C₁- C₄ alkyl sulfénylamino.

A est un atome de soufre ou d'oxygène
De préférence, les substituants Z admissibles sont choisis parmi les substituants suivants:
les atomes d'halogène (I,Cl,Br,F); les radicaux C₆- C₁₀ aryle éventuellement substitués par 1 à 6 substituants choisis parmi les atomes d'halogène (I,Cl,Br,F), les radicaux C₁- C₆ alkyle, C₁- C₆ alkoxy, polyhalo C1- C6 alkyle, polyhalo C₁- C₆ alkoxy; les radicaux C₁- C₉ hétéroaryle comportant en outre 1 à 4 hétéro atomes choisis parmi l'azote, le soufre, l'oxygène éventuellement substitué par l'un des substituants précédemment définis pour les radicaux C₆- C₁₀ aryle ; les radicaux C₁- C₆ alkoxy ; polyhalo C₁- C₆ alkoxy, cyano, amino; C₁- C₆ alkyle; polyhalo C₁- C₆ alkyle.

Dans le cas où R est un radical carbocyclique ou hétérocyclique, les substituants Z sont les mêmes que pour les radicaux carboacycliques et en plus peuvent correspondent aux radicaux C₁- C₆ alkyle ; polyhalo C₁-C₆ alkyle.

De préférence, dans ce dernier cas, les substituants sont les mêmes que pour les substituants préférés des radicaux carboacyclique mais en plus peuvent correspondre aux radicaux C₁- C₆ alkyle ; polyhalo C₁- C₆ alkyle.

Normalement les radicaux R comptent 0 à 6 substituants Z et dans le cadre de cette description le terme polyhalo correspond à 1 à 6 atomes d'halogène. De plus lorsque ce n'est pas spécifié autrement les radicaux alkyle en général (y compris alkoxy, alkylthio, etc..) sont linéaires ou ramifiés
Les groupes acycliques comportent 1 à 24 atomes de carbone, de préférence.

De préférence, les systèmes monocycliques peuvent être représentés par la formule 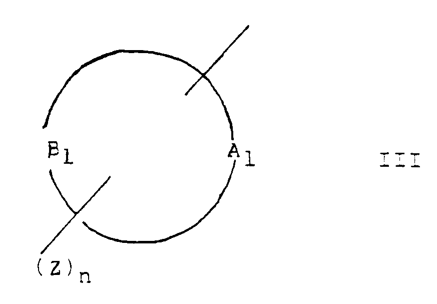
dans laquelle B₁ représente un carbone saturé ou insaturé et A₁ représente une chaîne d'atome qui ensemble avec B₁ forme un système monocyclique contenant de 0 à 3 double liaisons ou 0 à 2 triple liaisons. A₁ peut comprendre 2 à 12 atomes de carbone ou peut comprendre une combinaison de 1 à 11 atomes de carbone et 1 à 4 hétéroatomes qui peuvent être choisis indépendamment parmi N, O, S, P ou un autre hétéroatome ou peut contenir 4 hétéroatomes seuls formant un cycle avec B₁.

Les systèmes comprenant des hétéroatomes peuvent dans certains cas porter des atomes d'oxygène comme dans les systèmes aromatiques contenant un groupe N-oxyde ou contenant un groupe sulfinyl, sulfonyl, selenoxyde et phosphine oxyde.

Certains carbones des cycles formés par A₁ et B₁ peuvent porter des groupements carbonyl, thiocarbonyl, méthylidène, oxime ou imino éventuellement substitués par un groupes C₁- C₆ alkyle, C₃- C₈ cycloalkyle, C₆- C₁₀ aryle lesdits groupes étant éventuellement substitués par un à 6 groupements Z tels que définis précédemment.

Le groupe désigné par Z représente un ou plusieurs substituant choisis indépendémment parmi le groupe de substituant définis précédemment pour Z. En général n = 0 à 6.

Les systèmes bicycliques peuvent être représentés par les formules : 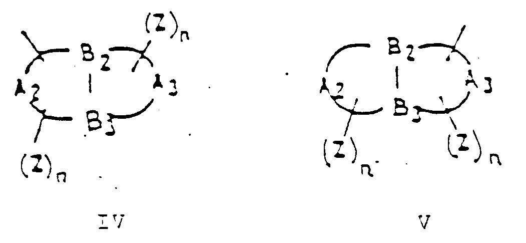
dans lesquelles B₂ et B₃ peuvent être indépendamment un atome de carbone saturé ou insaturé ou un atome d'azote, A₂ et A₃ indépendamment représentent une chaîne d'atomes décrite ci- dessous et Z représente un ou plusieurs substituants sélectionnés indépendamment parmi le groupe de substituants définis précédemment pour Z. Les groupes A₂ et A₃ peuvent contenir en combinaison avec B₂ ou B₃ de 0 à 5 doubles liaisons. A₂ et A₃ indépendamment de B₂ et B₃ peuvent contenir de 1 à 3 hétéroatomes qui peuvent être choisis parmi N, O, S, P ou d'autres hétéroatomes ensemble avec 1 à 10 atomes de carbone ou peuvent contenir de 1 à 3 hétéroatomes seuls formant le cycle.

Les hétéroatomes peuvent dans certains cas porter des atomes d'oxygène comme dans les cycles aromatiques N- oxyde et des systèmes contenant des groupes sulfinyl, sulfonyl, selenoxyde et oxyde de phosphine. Certains atomes de carbone peuvent être des groupes carbonyl, thiocarbonyl, des groupes imine ou méthylidène ou oxime ces groupes étant éventuellement substitués par un groupe C₁- C₁₂ alkyle, C₃- C₈ cycloalkyle, C₆- C₁₀ aryle éventuellement substitués par un à 6 groupement Z tels que définis précédemment.

Les groupes Z dans les formules IV et V sont identiques ou différents et leur nombre est tel que n est compris entre 0 et 6 (n étant identique ou différent pour chaque cycle).

En ce qui concerne les structures englobés par IV et V, on doit noter que :
a) Quand B₂ et B₃ correspondent à l'atome d'azote, les groupes A₂ et A₃ ne doivent pas contenir moins de 3 atomes chacun.
b) Quand B₂ mais non B₃ est l'atome d'azote soit A₂ soit A₃ devraient contenir au moins 3 atomes et l'autre au moins deux.
c) Quand soit A₂ ou A₃ contiennent moins de 3 atomes, l'autre contient au moins 3 atomes et le pont doit être saturé
d) Quand le groupe A₂ ou A₃ contient un atome de carbone portant un carbonyl, thionyl, imino ou méthylidène ou oxime, il doit former ensemble avec B₂ et B₃ un cycle ayant au moins quatre membres.
e) Quand une double liaison annulaire est exocyclique à l'un des deux cycles elle doit être incluse dans un cycle contenant au moins cinq membres ou être exocyclique au cycle contenant au moins cinq membres.
f) Quand un chaînon A₂ ou A₃ est attaché aux atomes du pont B₂ et B₃ par deux doubles liaisons, le groupe A₂ ou A₃ est compris pour inclure une double liaison et les atomes du pont sont considérés comme étant insaturés.

Il doit être compris que les systèmes bicycliques peuvent être spirocycliques.

Les systèmes polycycliques ayant plus de deux cycles peuvent être représentés par les formules : 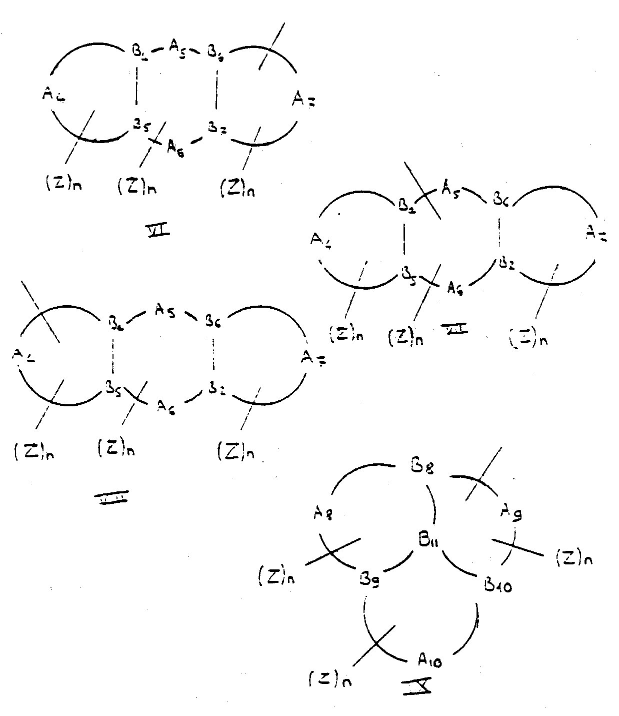
dans lesquelles B₄, B₅, B₆ et B₇ peuvent être indépendamment un atome de carbone saturé ou insaturé ou un atome d'azote saturé et A₄, A₅, A₆ et A₇ indépendamment représentent des chaînes d'atomes qui peuvent contenir ensemble avec l'un ou l'autre, (mais pas les deux) de leur atomes pontés associés de 0 à 2 doubles liaisons. Les groupes Z sont identiques à ceux précedemment indiqués.

A₄, A₅, A₆ et A₇ indépendamment de B₄, B₅, B₆ et B₇ peuvent contenir de 1 à 11 atomes de carbone ou pourrait contenir une combinaison de 1- 10 atomes de carbone et de 1 à 3 hétéroatomes sélectionnés indépendamment parmi N, O, S, P ou autres hétéroatomes ou pourrait contenir 1 à 3 hétéroatomes seuls.

Les hétéroatomes peuvent dans certains cas porter des atomes d'oxygène comme dans les cycles aromatiques N- oxyde et des systèmes contenant des groupes sulfinyl, sulfonyl, selenoxyde et oxyde de phosphine. Certains atomes de carbone peuvent être des groupes carbonyl, thiocarbonyl, des groupes imine ou méthylidène ou oxime éventuellement substitués par un groupe C₁- C₁₂ alkyle, C₃- C₈ cycloalkyle, C₆- C₁₀ aryle ces groupes étant éventuellement substitués par un à six groupements Z tels que définis précédemment.

Les groupes Z dans les formules VI à IX sont dentiques ou différents et leur nombre est tel que n est compris entre 0 et 6 (n étant identique ou différent pour chaque cycle). Concernant la structure IX, les groupes B₈, B₉ et B₁₀ représentent indépendamment un atome de carbone saturé ou insaturé ou un atome d'azote saturé.

Le groupe B₁₁ peut représenter un atome de carbone saturé ou insaturé ou un atome d'azote ou de phosphore.

Les groupes A₈, A₉, A₁₀ représentent des chaînons d'atome qui peuvent contenir ensemble avec 1 des groupes B₈, B₉, B₁₀ et B₁₁ de 0 à 2 doubles liaisons. Les chaînons des groupes A₈, A₉, A₁₀ indépendamment des groupes B₈, B₉, B₁₀, et B₁₁ peuvent contenir de 2 à 10 atomes de carbone ou 1 à 10 atomes de carbone en combinaison avec 1 à 3 hétéroatomes choisis parmi N, O, S, P ou autres ou peuvent contenir de 2 à 3 hétéroatomes seuls.Les hétéroatomes peuvent dans certains cas porter des atomes d'oxygène comme dans les cycles aromatiques N- oxyde et des systèmes contenant des groupes sulfinyl, sulfonyl, selenoxyde et oxyde de phosphine. Certains atomes de carbone peuvent être des groupes carbonyl, thiocarbonyl, des groupes imine ou méthylidène ou oxime, ces groupes étant éventuellement substitués par un groupe C₁- C₁₂ alkyle, C₃- C₈ cycloalkyle, C₆- C₁₀ aryle éventuellement substitués par un à six groupements Z tels que définis précédemment.

Les groupes Z dans la formule IX sont identiques ou différents et leur nombre est tel que n est compris entre 0 et 6 (n étant identique ou différent pour chaque cycle).

On doit noter que les groupes polycycliques peuvent être spirocycliques saturés ou insaturés et éventuellement substitués par un ou plusieurs substituants Z précédemment indiqués et n = 1 à 6 avec la précision que n est identique ou différent pour chaque cycle.

Les systèmes bicycliques pontés peuvent être représentés par les formules généralisées 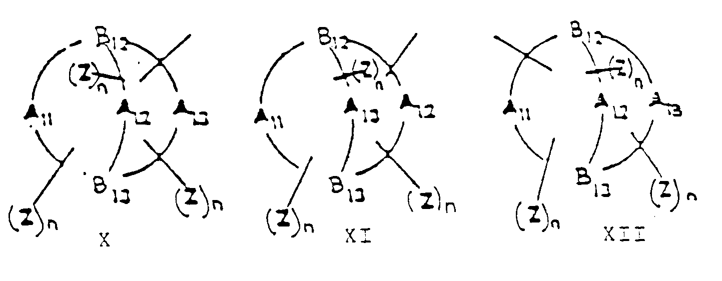
dans lesquelles B₁₂ et B₁₃ peuvent être indépendamment un atome de carbone saturé éventuellement substitué par un des groupes Z ou un atome d'azote et les groupes A₁₁, A₁₂ et A₁₃.indépendamment représentent des chaînons d'atomes qui peuvent contenir indépendamment de B₁₂ et B₁₃ de 0 à 2 doubles liaisons
Les chaînons A₁₁, A₁₂, A₁₃ indépendamment de B₁₂ et B₁₃ peuvent contenir de 1- 11 atomes de carbones ou 1 à 10 atomes de carbone et 1- 3 hétéroatomes qui peuvent être choisis indépendamment parmi N, O, S, P ou autres ou peuvent contenir 1- 3 hétéroatomes seuls avec la condition que quand un des chaînons A₁₁, A₁₂ et A₁₃ sont un seul hétéroatome les deux autres chaînons doivent comprendre au moins 2 atomes, une seconde condition étant que , quand un ou les deux chaînons B₁₂ et B₁₃ sont l'atome d'azote, les chaînons A₁₁, A₁₂ et A₁₃ doivent comprendre au moins deux atomes saturés.

Les hétéroatomes peuvent dans certains cas porter des atomes d'oxygène comme dans les cycles aromatiques N- oxyde et des systèmes contenant des groupes sulfinyl, sulfonyl, selenoxyde et oxyde de phosphine. Certains atomes de carbone peuvent être des groupes carbonyl, thiocarbonyl, des groupes imine ou méthylidène ou oxime ces groupes étant éventuellement substitués par un groupe C₁- C₁₂ alkyle, C₃- C₈ cycloalkyle, C₆- C₁₀ aryle éventuellement substitués par un à six groupement Z tels que définis précédemment.

Les groupes Z dans les formules X, XI et XII sont identiques ou différents et leur nombre est tel que n est compris entre 0 et 6 (n étant identique ou différent pour chaque cycle
Ce procédé est particulièrement adapté à l'obtention de composés à partir de disulfures où R est un radical phényle eventuellement substitué par un ou plusieurs radicaux choisis parmi les radicaux suivants:
les atomes d'halogène (I,Cl,Br,F); les radicaux C₆- C₁₀ aryle éventuellement substitués par 1 à 6 substituants choisis parmi les atomes d'halogène (I,Cl,Br,F), les radicaux C₁- C₆ alkyle, C₁- C₆ alkoxy, polyhalo C₁- C₆ alkyle, polyhalo C₁- C₆ alkoxy; les radicaux C₁- C₉ hétéroaryle comportant en outre 1 à 4 hétéro atomes choisis parmi l'azote, le soufre, l'oxygène éventuellement substitué par l'un des substituants précédemment définis pour les radicaux C₆- C₁₀ aryle ; les radicaux C₁- C₆ alkoxy ; polyhalo C₁- C₆ alkoxy, les radicaux C₁- C₆ alkyle ou C₁- C₆ polyhaloalkyle, cyano, amino.

Ce procédé est particulièrement adapté à l'obtention de composés à partir de disulfures où R est un radical alkyle éventuellement substitué par un ou plusieurs radicaux choisis parmi les radicaux suivants:
les atomes d'halogène (I,Cl,Br,F); les radicaux C₆- C₁₀ aryle éventuellement substitués par 1 à 6 substituants choisis parmi les atomes d'halogène (I,Cl,Br,F), les radicaux C₁- C₆ alkyle, C₁- C₆ alkoxy, polyhalo C₁- C₆ alkyle, polyhalo C₁- C₆ alkoxy; les radicaux C₁- C₉ hétéroaryle comportant en outre 1 à 4 hétéro atomes choisis parmi l'azote, le soufre,
l'oxygène éventuellement substitué par l'un des substituants précédemment définis pour les radicaux C₆-C₁₀ aryle ; les radicaux C₁- C₆ alkoxy ; polyhalo C₁- C₆ alkoxy, cyano, amino.

Ce procédé est particulièrement adapté à la préparation de pyrazoles dans laquelle R répond à la formule : 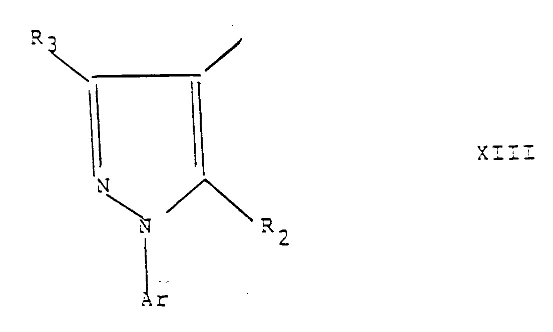
R₂ représente un groupe amino NHR₄R₅ dans lequel R₄, R₅ identiques ou différents représentent un atome d'hydrogène ; un groupe C₁-C₆ alkyle éventuellement substitué par un groupe C₂-C₅ alkoxycarbonyle ; C₃-C₆ cycloalkyle; C₂- C₇ alcanoyle éventuellement formant ensemble un groupe imide cyclique de 5 à 6 atomes avec l'atome d'azote auquel ils sont attachés, lesdits groupes pouvant être éventuellement substitués par un à six atomes d'halogène ; C₂-C₇ alkoxycarbonyle ; polyhalo C₂-C₇ alkoxycarbonyle ; R₂ représente encore un groupe C₁-C₄ alkylsulfénylamino ; C₂- C₅ alkoxyméthylènamino éventuellement substitué sur le méthylène par un groupe C₁- C₄ alkyle ; un atome d'halogène ; un groupe C₁- C₆ alkyle ; carboxy ; C₁-C₆ alkylthio ; polyhalo C₁- C₆ alkylthio ; C₁- C₆ alkylsufinyle ; polyhalo C₁- C₆ alkylsulfinyle, C₁- C₆ alkylsulfonyle, polyhalo C₁- C₆ alkylsulfonyle; tri C₁- C₆ alkylsilylmethyl ; tri C₁- C₆ alkylsilyl ; cyano ; hydroxy ; hydroxy C₁- C₆ alkylamino, C₁- C₆ alkoxy
R₂ représente encore l'atome d'hydrogène, un reste HN- C(=A)- R₆, R₆ étant un atome d'hydrogène, un radical C₁- C₆ alkyle ; C₂- C₄ alkenyle ; C₂- C₄ alkinyle ; C₁- C₄ alkoxyalkyle, C₁- C₄ alkylthioalkyle, C₁- C₄ alkoxy ; C₁- C₄ alkylthio, C₁- C₄ alkylamino, di(C₁- C₄ alkyl)amino ; polyhalo C₁- C₄ alkyle ; C₃- C₇ cycloalkyl éventuellement substitué par un ou plusieurs atomes d'halogène, C₁- C₄ alkyle ; C₁- C₄ halogènoalkyl ou R₆ représente encore un noyau phényl ; phénylthio ; phénoxy ; phénylamino, ces noyaux phényl étant éventuellement substitués par les radicaux cyano, C₁- C₄ alkyl, C₁- C₄ alkoxy ; C₁- C₄ alkylthio ; C₁- C₄ alkylsulfinyle ; C₁- C₄ alkylsulfonyle ; polyhalo C₁-C₄ alkyle ; polyhalo C₁- C₄ alkoxy ; polyhalo C₁-C₄ alkylthio ; polyhalo C₁- C₄ alkylsulfinyle ;polyhalo C₁-C₄ alkylsulfonyle ; les atomes d'halogène.
A est un atome de soufre ou d'oxygène
R₃ est NO₂, CO₂ C1- C6 alkyl, un atome d'halogène ; d'hydrogène ; un groupe cyano ou C₁- C₆ alkyle ; polyhalo C₁- C₆ alkyle ; C₃- C₆ cylcloalkyle
Ar est un noyau phényle ou pyridile éventuellement substitué par 1 à 4 substituants choisis parmi les radicaux cyano, C₁- C₄ alkyl, C₁- C₄ alkoxy ; C₁- C₄ alkylthio ; C₁- C₄ alkylsulfinyl ; C₁- C₄ alkylsulfonyl ; polyhalo C₁-C₄ alkyl ; polyhalo C₁-C₄ alkoxy ; polyhalo C₁- C₄ alkylthio ; polyhalo C₁- C₄ alkylsulfinyl ;polyhalo C₁- C₄ alkylsulfonyl ; les atomes d'halogènes,
Ces derniers disulfures pyrazoles sont décrits dans les demandes de brevet européen n° 0201852 et 0234119.

L'homme de métier, pour la préparation de ces disulfures pourra utilement se reporter à la description de ces deux documents.

Ce procédé est très avantageusement adapté pour la préparation de composés dans lesquels
R répond à la formule 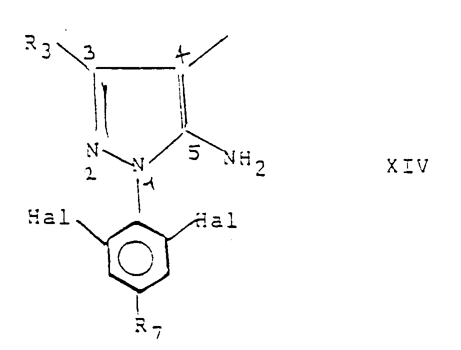
dans laquelle :
R₃ a la même signification que précédemment
Hal est un atome de fluor, de brome ou de chlore ou est un atome d'hydrogène
R₇ est un groupe trifluorométhyl ou trifluorométhoxy et aussi un halogène.

Parmi les composés de formule (II), on préfère utiliser le bromure de perfluoroalkyle lorsque la chaîne alkyle contient un seul atome de carbone et les iodures de perfluoroalkyle lorsque la chaîne alkyle contient au moins deux atomes.

En effet, le bromure de trifluorométhyle est un gaz extincteur (M.R.C. "GERSTENBERGER, A. HASS Angew. Cem. Int." Ed 1981, 20, 647) qui est un produit industriel fabriqué à large échelle, donc d'un coût tout à fait accessible pour l'industrie. L'iodure de trifluorométhyle n'étant pas industriel n'est disponible qu'à un prix qui le rend difficilement utilisable. Par contre, dès que la chaîne alkyle a au moins 2 atomes, les iodures de perfluoroalkyle se présentent sur le marché à des prix nettement inférieurs à ceux de leurs homologues bromés.

Le solvant choisi doit, autant que possible, permettre de solubiliser le dithionite ou l'hydroxyméthanesulfinate et l'halogénure de perfluoroalkyle.

Répondent à cette condition, les solvants polaires et parmi eux préférentiellement :
le formamide
le diméthylformamide (D.M.F.)
le diméthylacétamide (D.M.A.)
l'hexaméthylphosphoramide (H.M.P.A.)
la N- méthylpyrrolidone (N.M.P.)
le diméthylsulfoxide (D.M.S.O)
le sulfolane
les éthers tels que le dioxanne,
le tétrahydrofuranne, le diméthoxyéthane.

Parmi les amides on préfère tout particulièrement utiliser le diméthylformamide.

On appelle réducteur, les agents susceptibles de provoquer la formation de radicaux libres CFYT à partir de XCFYT.

Selon une première variante on utilisera comme agents les métaux choisis parmi le groupe constitué par le zinc, le cadmium, l'aluminium, le manganèse, en mélange avec du dioxyde de soufre, les dithionites et les hydroxyméthane sulfinates.

Parmi les métaux objet du procédé selon l'invention, on préfère utiliser le zinc.

Le dithionite alcalin, alcalino terreux ou métallique répond de préférence à la formule générale (XV) Mn(S₂O₄) dans laquelle n est égal à 1 ou 2 suivant la valence de métal M.

Parmi les composés de formule (XV), on préfère utiliser le dithionite de sodium ou de potassium. On préfère tout particulièrement utiliser le dithionite de sodium.

Parmi les hydroxyméthanesulfinates, on préfère utiliser l'hydroxyméthanesulfinate de sodium (plus connu sous le nom commercial de Rongalite) ou l'hydroxyméthanesulfinate de zinc (plus connu sous le nom commercial de Decroline)
Lorsque l'on utilise un dithionite de formule générale (XV) ou un hydroxyméthanesulfinate on ajoute avantageusement un base choisie parmi les hydroxydes alcalins ou alcalino terreux, l'ammoniaque, la trisdioxa- 3,6 heptylamine, le chlorure de triéthylbenzylammoniumn, les sels d'acides faibles tels que par exemple le phosphate disodique, le métabisulfite de sodium, l'hydrogénosulfite de sodium, le borate de sodium. On préfère utiliser le phosphate disodique. La quantité de base utilisée varie avantageusement selon un rapport molaire calculé par rapport au disulfure compris entre 0,3 et 3.

La quantité molaire de zinc ou de dithionite ou d'hydroxyméthanesulfinate utilisée par rapport au disulfure est notamment supérieure à 1 et plus préférentiellement.comprise entre 1 et 3.

Selon une seconde variante qui est également la variante préférée on utilisera le mélange SO₂ anion formiate
Les anions formiate proviennent avantageusement des formiates de formule :

(HCOO-)n R₁ n+

R₁ n+ , n étant égal à 1 ou 2, étant choisi parmi les cations de métal alcalin (Na, K, Li), alcalino-terreux (Ca), ammonium de formule NR₂R₃R₄R₅, R₂, R₃, R₄, R₅, étant choisis parmi l'atome d'hydrogène, les radicaux C₁-C₁₈ alkyle, C₂-C₁₈ alcényle, C₂-C₁₈ alcynyle, lesdits radicaux étant éventuellement substitués par un radical hydroxy.

De préférence, R₁ n+ est choisi parmi les cations de métal alcalin, notamment le sodium, l'ammonium, l'isopropyl ammonium, le triéthylammonium, le triméthylammonium, le ter-butyl ammonium, l'éthanolammonium
La quantité molaire de formiate utilisée par rapport au disulfure est notamment supérieure à 1 et plus préférentiellement comprise entre 1 et 5.

Le dioxyde de soufre peut être présent en quantité catalytique. La proportion molaire de SO₂ par rapport au formiate est généralement comprise entre O.O1 et 4 bien que la limite supérieure ne soit pas critique.

Pour une meilleure mise en oeuvre de l'invention lorsque l'halogénure de formule II est liquide ou solide, la quantité molaire de ce dernier mise en oeuvre par rapport au disulfure est notamment supérieure ou égale à 1 et de préférence comprise entre 1 et 3.

Lorsque l'halogénure de formule II est gazeux comme c'est le cas pour CF₃Br, la quantité molaire de ce dernier mise en oeuvre par rapport au disulfure est notamment supérieure à 1
Selon un premier procédé de mise en oeuvre de l'invention, lorsque l'on utilise un métal, celui- ci est utilisé en poudre ou en tournure et le dioxyde de soufre est avantageusement introduit sous forme gazeuse avant l'introduction de l'halogénure de formule (II).

Selon un deuxième procédé de mise en oeuvre de l'invention lorsque l'on utilise un dithionite alcalin, celui- ci est introduit dans le réacteur en solution saturée dans l'eau ou le formamide.Il est même possible d'introduire le dithionite sous forme solide. On préfère éliminer tout l'oxygène présent dans le réacteur puis éventuellement on introduit du dioxyde de soufre et on introduit le perhalogénoalkane.

Selon un troisième procédé de mise en oeuvre de l'invention lorsque l'on utilise un hydroxyméthanesulfinate, on introduit ce dernier directement sous forme solide dans le solvant réactionnel.

On introduit éventuellement du dioxyde de soufre puis le perhalogénoalkane.

Selon un procédé de mise en oeuvre de l'invention, on introduit successivement le disulfure puis le formiate, le solvant, le SO₂ sous forme gazeuse puis le perhalogénoalcane.

A la fin de la réaction on sépare le ou les solvants et les produits réactionnels puis on purifie le composé perhalogénoalkylé, par exemple, par extraction à l'aide de solvants tels l'éther éthylique ou les éthers de pétrole.

En ce qui concerne les conditions de réaction, il est préférable de travailler à une température comprise entre 20° et 100 °C ou température d'ébullition du solvant et encore plus préférentiellement lorsqu'on utilise un dithionite à une température comprise entre 20° et 80°C.

Dans le cas préféré d'un halogénure gazeux comme le CF₃Br il est néanmmoins avantageux de faire la réaction dans un milieu solvant qui dissout l'halogénure au moins faiblement à pression atmosphérique et de façon beaucoup plus importante sous pression. C'est le cas par exemple du diméthylformamide pour CF₃Br
Lorsque l'on travaille avec un gaz peu soluble dans le solvant de réaction, la pression réactionelle est généralement supérieure à 1 bar. Une pression comprise entre 1 et 50 bars est préférée bien que la limite supérieure ne soit pas un critère essentiel mais uniquement préférentiel du point de vue technique.

Ainsi la pression réactionelle est généralement supérieure à 1 bar (pression du gaz halogénure). Sur un plan industriel une pression comprise entre 1 et 50 bars est préférée bien que la limite supérieure ne soit pas un critère essentiel mais uniquement préférentiel d'un point de vue technique.

D'une manière générale, lorsque l'halogénure de formule II est gazeux, il est avantageux de conduire la réaction dans des conditions de pression et température subcritiques.

De préférence le réacteur ne doit pas être constitué d'un matériau réactif tel que ceux décrits dans la demande de brevet publiée sous le numéro EP 165 135. On préfère ainsi utiliser un réacteur en verre.

On peut citer parmi les produits obtenus par le procédé de la présente invention
le trifluorométhylthiobenzène,
le benzyltrifluorométhylsulfure,
le méthyltrifluorométhylsulfure,
le méthylperfluorooctylesulfure,
le trifluorométhylthioacétate d'éthyle et
le butylperfluorobutylsulfure,
le 4- trifluorométhylthio 3- cyano 5- amino,
1- (2,6- dichloro 4- trifluorométhylphenyl) pyrazole.

Les composés objets du procédé de l'invention sont notamment utilisés comme intermédiaires de synthèse dans l'industrie pharmaceutique ou phytosanitaire.

Les disulfures de départ sont obtenus de manière connue.

L'invention va être plus complètement décrite à l'aide des exemples suivants qui ne devront pas être considérés comme limitatifs de la présente invention.

### EXEMPLE 1 :

Dans un flacon en verre épais, on introduit 30 ml de diméthylformamide, 15 ml d'eau, 10 g de dithionite de sodium, 10 g d'hydrogénophosphate de sodium, 5,5 g de phényldisulfure, on fait le vide dans le flacon puis on le thermostate à 20°C, ensuite le flacon est agité pendant 6 heures sous une pression de bromotrifluorométane comprise entre 5 et 2,5 atmosphères. On ajoute 100 ml d'eau et on extrait à l'éther. Après lavage avec 2 fois 20 ml d'acide chlorhydrique à 5 %, puis avec du carbonate de sodium à 10%, la phase éthérée est séchée sur sulfate de magnésium.

Après évaporation du solvant, on obtient le trifluorométhylthiobenzène avec 65 % de rendement.
Eb : 77°C/20 mm Hg F = - 42 ppm

### EXEMPLE 2 :

Dans le même flacon qu'à l'exemple 1, on place30 ml de diméthylformamide, 6,5 g de zinc, 4 g de dioxyde de soufre, 5,5 g de phényldisulfure. Après réaction à 20°C et traitement habituel, on obtient le trifluorométhylthiobenzène.

### EXEMPLE 3 :

Dans le même flacon qu'à l'exemple 1, on place 30 ml de diméthylformamide, 2 g d'eau, 15,5 g d'hydroxyméthanesulfinate de sodium et 5,5 g de phényldisulfure. Après réaction à 20°C et traitement habituel, on obtient le trifluorométhylthiobenzène avec 93 % de rendement.

### EXEMPLE 4 :

Dans le même flacon qu'à l'exemple 1, on place 30 ml de diméthylformamide, 2 g d'eau, 13 g d'hydroxymétanesulfinate de zinc et 5,5 g de phényldisulfure. Après réaction à 20°C et traitement habituel, on obtient le trifluorométhylthiobenzène.

### EXEMPLE 5 :

On répète l'expérience 3 en remplaçant le phényldisulfure par 5,9 g de dithioacétate d'éthyle.Après réaction à 20°C et traitement habituel on obtient le trifluorométhylthioacétate d'éthyle avec 55 % de rendement.
Eb 71°C/100 mm Hg F = - 41,7 ppm H = 4,27 ppm (2 H, q, 3,73 ppm (2 H, s), 1,3 ppm (3 H, t).

### EXEMPLE 6 :

On répète l'expérience 5 avec 4,5 g de butyldisulfur et on obtient le butyltrifluorométhylsulfure avec 31 % de rendement.
Eb 95°C F = - 41 ppm H (CH₂S) 2,7 ppm.

### EXEMPLE 7 :

3,5 g d'iodure de perfluorobutyle, 4 g d'hydroxyméthanesulfinate de sodium, 2;5 g de benzyldisulfure dans 10 ml de diméthylformamide et 0,5 ml d'eau sont agités pendant 6 heures. Après traitement habituel, on obtient le benzylperfluorobutylsulfure, rendement 17 %
Eb 92°C/17mm Hg F (CF₂S) = - 88,8 ppm H 7,3 ppm (5H, s) 4,2 ppm (2H, s).

### EXEMPLE 8 :

5,5 g d'iodure de perfluorooctyle, 3 g de dithionite de sodium, 3 g d'hydrogénophosphate de sodium, 1 g de méthyldisulfure sont agitées dans 10 ml de diméthylformamide et 5 ml d'eau pendant 6 heures. Après traitement habituel on obtient le méthylperfluorooctylsulfure avec 20 % de rendement
Eb 44°C/10 mm Hg H : 2,4 ppm (s) F (CF₂) : - 92,3 ppm.

### EXEMPLE 9 :

4,5 g d'iodure de perfluorohexyle, 4 g d'hydroxyméthanesulfinate de sodium et 2,2 g de phényldisulfure dans 10 ml de diméthylformamide et 0,5 ml d'eau sont agités pendant 12 heures. Après traitement habituel, on obtient le phénylperfluorohexylsulfure avec 40 % de rendement
F (CF₂) - 87,2 ppm ES 99°C/18 mm Hg.

### EXEMPLE 10 :

On remplace dans l'exemple 9 le phényldisulfure par 1,8 g de butyldisulfure, on obtient ainsi le butylperfluorohexylsulfure avec 22 % de rendement
F = - 86,3 ppm (SCF₂) H = 2,7 ppm (CH₂S).

### EXEMPLE 11 :

On remplace dans l'exemple 10 l'hydroxyméthanesulfinate de sodium par 3,5 g du sel de zinc. Le produit est alors obtenu avec 16 % de rendement.

### EXEMPLE 12 :

On répète l'expérience 3 en remplaçant le bromotrifluorométhane par du dichlorodifluorométhane. Après réaction on obtient le chlorodifluorométhylthiobenzène.

### EXEMPLE 13 :

On répète l'expérience 3 en remplaçant le bromotrifluorométhane par du bromochlorodifluorométhane sous une pression de 1,7 atmosphère. Après réaction, on distille le chlorodifluorométhylbenzène
Eb 71°C/25 mm Hg F = - 27 ppm
Rendement 72 %

### EXEMPLE 14 :

On répète l'expérience 5 en remplaçant le bromotrifluorométhane par du bromochlorodifluorométhane.Après réaction, on obtient le chlorodifluorométhylthioacétate d'éthyle
Eb 81°C/25 mm Hg F = - 27 ppm H =4,23 ppm (2H,q, J = 10,5 Hz) 3,75 ppm (2H, s) 1,3 ppm (3H, t)
IR =1718 Cm⁻¹ Rendement 65 %

### EXEMPLE 15 :

### Préparation de 4- trifluorométhylthio 3- cyano 5-amino 1- (2,6- dicloro 4- trifluorométhylphényl) pyrazole

D'une part 2 g de disulfure de 5- amino 3-cyano 1- (2,6- dichloro 4- trifluorométhylphényl) 4-pyrazolyl sont dissous dans 120 ml de diméthylformamide ; d'autre part 3,05 g de hydrogénophosphate de sodium 12H₂O sont dissous dans 60 ml d'eau distillée. La solution de diméthylformamide est ensuite introduite dans un autoclave téfloné de 500 cm3, suivie de la solution aqueuse. 1,48 g de dithionite de sodium sont introduits à leur tour sous agitation.L'autoclave est alors fermé et CF₃Br introduit avec une pression de 12- 13 bars (pression autogène)
Après 2h 30 de bonne agitation (1000 tr/mn turbine rushton) à 25°C, nous obtenons les résultats suivants
TT = 100 %
RR dosé (CLHP étalon externe) 75 %

### EXEMPLE 16 :

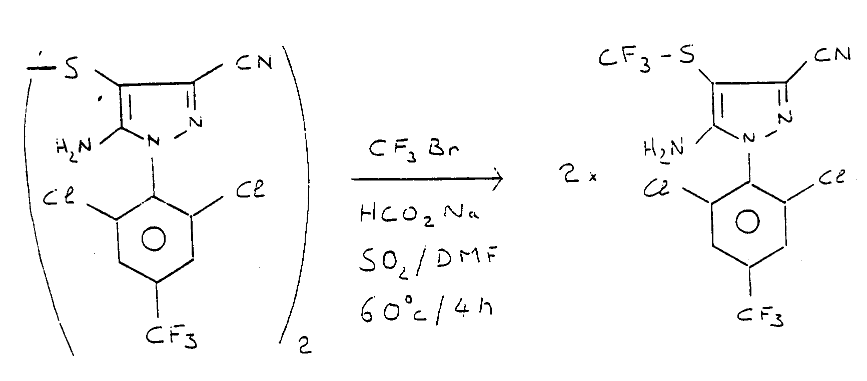

Dans un autoclave sont introduits successivement 4 g (5,7 mmoles), de pyrazole disulfure, 1,16 g (17,1 mmole) de formiate de sodium, 20 cm3 de DMF et 1,45 g (22,8 mmoles) de SO₂.

Ce mélange réactionnel bien agité est amené à une température de 60°C et une pression de CF₃Br de 13 bars est maintenue 4 h à cette température. L'analyse CLHP du milieu réactionnel conduit aux résultats suivants :
TT = 95 %
RR = 90 %
Le disulfure est fabriqué à partir du 5-amino-3-cyano-1-(2,6-dichloro -4-trifluorométhylphényl) -4-thiocyanatopyrazole obtenu selon la demande européenne n° 883053068 déposée le 10 juin 1988 au nom de May & Baker. On additionne à 3,0 g de ce produit dans 40 ml de chloroforme, 2 ml d'une solution à 50% de NaOH aqueux.

Le mélange est ensuite traité avec 100 mg de chlorure de tribenzylammonium et agité 6 heures à température ambiante. Le solide jaune est filtré, séché, purifié, élué sur colonne chromatographique avec un éluant dichlorométhane-acétate d'éthyle (4:1). Pour conduire à un solide jaune, qui est recristallisé à partir d'un mélange hexane-toluène pour fournir 1,59 g de cristaux jaunes, P F : 303-305 °C

### EXEMPLE 17

### Préparation de (dichloro-1,2 trifluoro-1,2,2 éthyl) phénylsulfure

3,8 g de trichloro-1,1,2 trifluoroéthane, 4,4 g de phényldisulfure, 7 g de dithionite de sodium et 6 g d'hydrogénophosphate de sodium sont agités dans 20 ml de diméthylformamide et 10 ml d'eau pendant 6 heures. Après entrainement à la vapeur et traitement habituel, on obtient le (dichloro-1,2 trifluoro-1,2,2 éthyl)phénylsulfure (1,7 g) avec 52 % de rendement
^{º} F : -63,3 ppm (2F,d,J=14,2 Hz) -89 ppm-t,1F), et le phénylthiotrifluoroéthylène avec 8% de rendement.

### EXEMPLE 18

### Préparation de (dichlorofluorométhyl)phénylsulfure

On répète l'expérience précédente avec 2,8 g de trichlorofluorométhane; on obtient le (dichlorofluorométhyl)phénylsulfure (0,55 g) avec 13% de rendement; ^{º}F : -18,7 ppm(s)

### EXEMPLE 19

### Préparation de (bromodifluorométhyl)phénylsulfure

On répète l'expérience précédente avec 4,6 g de dibromodifluorométhane, on obtient le (bromodifluorométhyl)phénylsulfure (0,3 g) avec 6% de rendement. ^{º}F: -19 ppm (s)

### EXEMPLE 20

### Préparation de 5-amino-4-(bromodifluorométhylthio)-3-cyano-1-(2,4,6-trichlorophényl)-1H-pyrazole

### Partie A : 5-amino-3-cyano-4-thiocyanato-1-(2,4,6-trichlorophényl)-1H-pyrazole

Une solution de 12,65 g (0,156 mole) de thiocyanate de sodium dans 62,5 ml de méthanol a été soumise à une agitation magnétique et a été refroidie à - 65°C dans un bain de neige carbonique-acétone. Puis une solution de 8,31 g (0,052 mole) de brome dans 62,5 ml de méthanol a été ajoutée avec soin à ce mélange en un temps d'approximativement 30 minutes, en maintenant la température dans l'intervalle de -65 à -60°C. Finalement, une suspension de 15,0 g (0,052 mole) de 5-amino-3-cyano-1-(2,4,6-trichlorophényl)-1H-pyrazole dans ml de méthanol a été ajoutée par portions au mélange sous agitation, tout en maintenant la température au-dessous de -47°C. Une portion supplémentaire de 50 50 ml de méthanol a été ensuite ajoutée pour rincer les cristaux de pyrazole restant dans le mélange. On a ensuite supprimé le refroidissement et on a laissé le mélange réactionnel se réchauffer à 18°C en 3,3 heures, temps au bout duquel le mélange a été entreposé au réfrigérateur à 0°C pendant une période de 16 heures. Puis on a laissé le mélange réactionnel se réchauffer à température ambiante en un temps de 2,5 heures et on l'a ensuite versé, sous agitation, dans 1000 ml d'eau pour précipiter le produit. Ce dernier a été recueilli par filtration sous vide, a été lavé à l'eau et séché à l'air.Une déshydratation a été effectuée par dissolution dans le dichlorométhane et mise en contact avec MgSO₄. Une filtration et une élimination du solvant par entraînement sous vide ont ensuite donné 16,2 g (90,4 %) de 5-amino-4-cyano-3-thiocyanato-1-(2,4,6-trichlorophényl)-1H-pyrazole sous forme d'une substance solide de couleur jaune clair.
Analyse : 1H-RMN (d6-DMSO) ^{º} 7,19 (s, 2H), 7,95 (s,2H) ppm.
IR (KBr) 2160, 2255 cm⁻¹.

### Partie B : 4,4′-dithiobis[5-amino-3-cyano-1-(2,4,6-trichlorophényl)-1H-pyrazole]

On a ajouté à une suspension sous agitation de 16,2 g (0,047 mole) de 5-amino-3-cyano-4-thiocyanato-1-(2,4,6-trichlorophényl)-1H-pyrazole, dans 180 ml de CHCl₃, 0,32 g (0,0014 mole) de chlorure de benzyltriéthylammonium et une solution de 6,0 g (0,15 mole) de NaOH dans 20 ml d'eau. Le mélange résultant a été agité sous l'atmosphère du laboratoire, à température ambiante, pendant 3,1 heures, temps au bout duquel une CCM d'une portion aliquote réactionnelle a indiqué que la réaction était achevée. Le produit solide de couleur jaune a été séparé par filtration, lavé à l'eau, puis dissous dans l'acétate d'éthyle, la solution étant soumise à une extraction avec 2x200 ml d'eau et à une déshydratation sur MgSO₄. Une déshydratation à l'étuve, sous vide, à une température de 70 à 75°C pendant approximativement 16 heures a donné 14,0 g (93,5 %) de 4,4′-dithiobis[5-amino-3-cyano-1-(2,4,6-trichlorophényl)-1H-pyrazole] sous forme d'une substance solide de couleur jaune.
Analyse :
1H-RMN (d6-DMSO) ^{º} 6,73 (s, 4H), 7,90 (s,4H) ppm.
IR (KBr) 1496, 1550, 1625, 2245 cm⁻¹.Partie C : 5-amino-4-(bromodifluorométhylthio)-3-cyano-1-(2,4,6-trichlorophényl)-1H-pyrazole
On a ajouté à une solution sous agitation de 1,0 g (0,0016 mole) de 4,4′-dithiobis [5-amino-3-cyano-1-(2,4,6-trichlorophényl)-1H-pyrazole], dans 10 ml de DMF, 0,42 g (0,0024 mole) de Na₂S₂O₄ et 0,34 g (0,0024 mole) de Na₂HPO₄, puis 5 ml d'eau et 1,01 g (0,0048 mole) de dibromodifluorométhane. Lorsque le mélange sous agitation est resté non homogène, une quantité supplémentaire de 15 ml de DMF et 5 ml de H₂O ont été ajoutés et la solution rapide résultante, contenant une petite quantité de substance semi-solide, a été ensuite agitée à température ambiante pendant 2,7 heures. Puis le mélange réactionnel a été agité avec 100 ml d'eau, plus 100 ml d'éther éthylique, et la phase éthérée a été séparée, déshydratée sur MgSO4 et filtrée. L'élimination de l'éther sous vide a donné 1,29 g d'une huile de couleur jaune qui a été introduite au sommet d'une colonne de chromatographie éclair contenant 65 g de gel de silice et a été éluée avec du dichloro-méthane, donnant 0,76 g (52,8 %) de 5-amino-4-(bromodi-fluorométhylthio)-3-cyano-1-(2,4,6-trichlorophényl)-1H-pyrazole sous forme d'une substance solide de couleur jaune pâle, P.F. 163,5-165°C.

| Analyse : C₁₁H₄BrCl₃F₂N₄S | | | |
|---|---|---|---|
| Calculé | C, 29,46 ; | H, 0,90 ; | N, 12,49 |
| Trouvé | C, 29,48 ; | H, 0,90 ; | N, 11,93 |

Spectre de masse en mode IE : m/z 448 (parent avec ³³Cl₂³⁷Cl⁷⁹Br et ³⁵Cl₃⁸¹Br), 319 (parent ³⁵Cl₂³⁷Cl⁷⁹Br moins CF₂⁷⁹Br).
Référence 132-DTM-7 : RPA 99428

### EXEMPLE 21

### Préparation de 5-amino-4-(bromochlorofluorométhylthio)-3-cyano-1-(2,4,6-trichlorophényl)-1H-pyrazole

On a ajouté à un mélange sous agitation de 3,0 g (0,0047 mole) de 4,4′-dithiobis[5-amino-3-cyano-1-(2,4,6-trichlorophényl)-1H-pyrazole] (préparé dans la partie B ci-dessus) et 75 ml de DMF 1,23 g (0,0070 mole) de Na₂S₂O₄, 1,0 g (0,0070 mole) de Na₂HPO₄, 30 ml d'eau et, finalement, 3,19 g (0,0141 mole) de chlorodibromofluorométhane et on a agité le mélange résultant à température ambiante pendant 40 minutes. Le mélange réactionnel a été versé dans 300 ml d'eau et la solution a été soumise à une première extraction avec de l'éther éthylique (1x300 ml), puis avec du dichlorométhane. Une chromatographie éclair sur colonne (gel de silice) de la matière obtenue en soumettant l'extrait éthéré à un entraînement sous vide a donné 1,05 g d'un produit relativement impur (Fraction 13A). L'élimination du dichlorométhane par entraînement sous vide a donné une fraction de produit contenant une grande quantité de N,N-diméthylformamide (DMF). Ce dernier a été éliminé par évaporation rotative à une température de 90 à 100°C en un temps de 2,5 heures, sous vide poussé, le résidu résultant étant dissous dans le dichlorométhane et soumis à une extraction avec de l'eau. L'élimination du solvant de la phase organique déshydratée et une chromatographie éclair sur colonne de gel de silice ont ensuite donné 0,50 g de 5-amino-4-(bromochlorofluorométhylthio)-3-cyano-1-(2,4,6-trichlorophényl)-1H-pyrazole sous forme d'une substance solide de couleur jaune brillant, P.F. 192-193°C. Le rendement total en produit, comprenant la fraction 13A, était égal à 71 %.

| Analyse : C₁₁H₄BrCl₄FN₄S | | | |
|---|---|---|---|
| Calculé | C, 28,42 ; | H, 0,87 ; | N, 12,05 |
| Trouvé | C, 28,63 ; | H, 0,86 ; | N, 11,92 |

Spectre de masse en mode IE : m/z 464 (parent avec ⁷⁹Br³⁵Cl₃³⁷Cl et ⁸¹Br³⁵Cl₄, 319 (parent ⁷⁹Br³⁵Cl₃³⁷Cl moins ⁷⁹Br³⁵ClFC).
Référence 132-DTM-21B : RPA 99466

### EXEMPLE 22

### Préparation de 5-amino-4-(bromochlorofluorométhylsulfinyl)-3-cyano-1-(2,4,6-trichlorophényl)-1H-pyrazole et de 5-amino-4-bromochlorofluorométhylsulfonyl)-3-cyano-1-(2,4,6-trichlorophényl)-1H-pyrazole

On a ajouté à un mélange sous agitation de 1,05 g (0,0023 mole) de 5-amino-4-bromochlorofluorométhylthio)-3-cyano-1-(2,4,6-trichlorophényl)-1H-pyrazole, obtenu sous forme de la fraction 13A dans l'exemple précédent, et 5 ml d'acide trifluoracétique, sous agitation et avec un refroidissement à 0°C, une solution de 0,42 ml (0,0041 mole) de H₂O₂ à 30 % dans 1 ml d'acide trifluoracétique. L'addition a été effectuée en un temps de 5 minutes au moyen d'une seringue insérée à travers une cloison en caoutchouc qui couvrait l'embouchure du ballon. Puis l'agitation a été poursuivie, en laissant la fusion progressive du bain de glace s'effectuer, pendant un temps approximatif de 18 heures. Le mélange réactionnel a été versé dans 30 ml d'eau, la substance solide résultante étant recueillie par filtration. La substance solide a été dissoute dans l'acétate d'éthyle, puis cette solution a été lavée avec une solution de NaHSO₃ à 10 % (2x25 ml), de la saumure (1x25 ml), une solution de NaHCO₃ saturée (2x25 ml) et a été ensuite soumise à une extraction finale avec de la saumure (2x25 ml). La phase organique a été déshydratée (MgSO₄), puis le solvant éliminé, donnant un résidu qui a été soumis à une chromatographie éclair sur une colonne de gel de silice, l'élution étant effectuée avec du dichlorométhane. Le sulfoxyde désiré a été recueilli dans les dernières fractions qui ont été réunies, débarrassées du solvant et déshydratées dans une étuve sous vide, donnant 0,39 g (35,0 %) de 5-amino-4-(bromochlorofluorométhyl-sulfinyl)-3-cyano-1-(2,4,6-trichlorophényl)-1H-pyrazole,
P.F. 225-226°C, avec décomposition.

| Analyse : C₁₁H₄BrCl₄FN₄OS | | | |
|---|---|---|---|
| Calculé | C, 27,47 ; | H, 0,84 ; | N, 11,65 |
| Trouvé | C, 27,82 ; | H, 0,86 ; | N, 11,21 |

Spectre de masse en mode IE : m/z 335 (parent ³³Cl₃³⁷Cl⁷⁹Br moins ⁷⁹Br³⁵ClFC).
Référence 132-DTM-29 : RPA 99568

### 5-amino-4-(bromochlorofluorométhylsulfonyl)-3-cyano-1-(2,4,6-trichlorophényl)-1H-pyrazole

Un nouveau traitement des fractions chromatographiques précédentes obtenues par la préparation de sulfoxyde décrite dans l'exemple précédent a donné, après évaporation sous vide du solvant, 0,12 g (10,5 %) de 5-amino-4-(bromochlorofluorométhylsulfonyl)-3-cyano-1-(2,4,6-trichlorophényl)-1H-pyrazole, P.F. 251,5-252,5°C, avec décomposition.

| Analyse : C₁₁H₄BrCl₄FN₄O₂S | | | |
|---|---|---|---|
| Calculé | C, 26,59 ; | H, 0,81 ; | N, 11,27 |
| Trouvé | C, 26,99 ; | H, 0,76 ; | N, 11,13 |

Référence 132-DTM-36 : RPA 99570

### EXEMPLE 23

### Préparation de 5-amino-4-(bromochlorofluorométhylthio)-3-cyano-1-[2,6-dichloro-4-(trifluorométhyl)phényl]-1H-pyrazole

On a ajouté à une solution sous agitation de 5,11 g (0,0073 mole) de 4,4-dithiobis [5-amino-3-cyano-1-[2,6-dichloro-4-(trifluorométhyl)phényl]-1H-pyrazole], dans 115 ml de DMF, 1,92 g (0,011 mole) de NA2S2O4 et 1,56 g de Na₂HPO₄, puis 45 ml d'eau, ce qui a provoqué une mise en solution partielle des réactifs inorganiques. Une portion de 4,96 g (0,0219 mole) de chlorodibromofluorométhane a été ensuite ajoutée, suivie par une quantité supplémentaire de 75 ml de DMF, avec pour résultat un milieu pratiquement homogène. Le mélange réactionnel a été agité à température ambiante pendant un temps de 1,6 heure, puis a été versé dans 450 ml d'eau et ce dernier mélange a été soumis à une extraction soigneuse avec une portion de 450 ml d'éther éthylique. La phase éthérée a été séparées déshydratée (MgSO₄) et les substances volatiles ont été éliminées sous une pompe à vide poussé à son maximum et à une température du bain de 100°C pour éliminer pratiquement la totalité du DMF. Le résidu a été soumis à une chromatographie éclair sur une colonne de gel de silice, en éluant avec CH₂Cl₂, et le produit recueilli a été déshydraté sous vide, donnant 1,76 g (24,2 %) de 5-amino-4-(bromochlorofluorométhylthio)-3-cyano-1-[2,6-dichloro-4-(trifluorométhyl)phényl]-1H-pyrazole,
P.F. 191,5-193°C.

| Analyse : C₁₂H₄BrCl₃F₄N₄S | | | |
|---|---|---|---|
| Calculé | C, 28,91 ; | H, 0,81 ; | N, 11,24 |
| Trouvé | C, 29,37 ; | H, 0,75 ; | N, 10,99 |

Spectre de masse en mode IE : m/z 498 (parent avec ³⁵Cl₂³⁷Cl⁷⁹Br et ³⁵Cl₃⁸¹Br), 351 (parent ³⁵Cl₂³⁷Cl⁷⁹Br moins ³⁷Cl⁷⁹BrFC).
Référence 132-DTM-31 RPA 99569

### EXEMPLE 24

### Préparation de 5-amino-4-(bromodifluorométhylthio-3-cyano-1-[2,6-dichloro-4-(trifluorométhyl)phényl]-1H-pyrazole

On a ajouté à une solution sous agitation de 1,94 g (0,003 mole) de 4,4′-dithiobis [5-amino-3-cyano-1-[2,6-dichloro-4-(trifluorométhyl)phényl]-1H-pyrazole, dans 45 ml de DMF, 0,78 g (0,0045 mole) de dithionite de sodium, 0,64 g de Na2HPO4 et 20 ml d'eau. Seule une solution partielle a été obtenue et des quantités supplémentaires de 30 ml de DMF et 5 ml d'eau ont été ajoutées, ce qui a provoqué la mise en solution de la plus grande partie des substances solides. Finalement, 1,89 g (0,009 mole) de CF₂Br₂ a été ajouté et le mélange résultant a été agité à température ambiante pendant un temps d'approximativement 17 heures. Le mélange réactionnel a été versé dans 185 ml d'eau, puis ce mélange a été soumis à une extraction soigneuse avec de l'éther éthylique. La phase éthérée séparée a été déshydratée sur MgSO4 et la totalité des substances volatiles a été éliminée par entraînement sous vide au moyen d'une pompe à la puissance maximale, sur un bain d'eau à 100°C, pendant plusieurs heures. Le résidu résultant a été soumis à une chromatograhie éclair sur une colonne de gel de silice, en éluant avec CH₂Cl₂, et le solvant a été évaporé, donnant 1,31 g (90,0 %) de 5-amino-4-(bromodifluorométhylthio-3-cyano-1-[2,6-dichloro-4-(trifluorométhyl)phényl]-1H-pyrazole sous forme d'une substance solide de couleur blanche, P.F. 162,5-163,5°C.

| Analyse : C₁₂H₄BrCl₂F₅N₄S | | | |
|---|---|---|---|
| Calculé | C, 29,90 ; | H, 0,84 ; | N, 11,62 |
| Trouvé | C, 30,03 ; | H, 0,75 ; | N, 11,39 |

Spectre de masse en mode IE : m/z 482 (parent, avec ³⁵Cl₂⁸¹Br et ³⁵Cl^{x37}Cl79Br), 351 (parent ³⁵Cl₂⁸¹Br moins CF₂⁸¹Br).
Référence 132-DTM-34 : RPA 99605.

### EXEMPLE 25

### Préparation de 5-amino-3-cyano-4-dichlorofluorométhylthio-1-(2,6-dichloro-4-trifluorométhylphényl) pyrazole.

On a ajouté de la poudre de zinc à une solution de bis [5-amino-3-cyano-1-(2,6-dichloro-4-trifluorométhylphényl) pyrazol-4-yl] disulfure (150 g) dans le diméthylformamide (1125 ml) et le mélange à été agité à température ambiante. On a ajouté ensuite à celui-ci une solution contenant 60,6 g de dioxyde de soufre dans 160 g de diméthyl- formamide suivi par 290 g de fluorotrichlorométhane. Après 30 mn environ un léger dégagement exothermique a été noté. La solution a été laissée agitée toute la nuit. On a filtré le mélange et on l'a versé lentement sur de la glace. Le solide récupéré a été lavé, séché et a donné un solide jaune/orange (185g) qui recristallisé dans un mélange toluène/hexane a donné un solide (123g) de point de fusion 187-189°C.

### EXEMPLE 26

### Préparation de 5-amino-3-cyano-4-dichlorofluorométhylthio-1-(2,6-dichloro-4-trifluorométhylphényl) pyrazole

Du phosphate de sodium dibasique a été dissout dans un mélange de diméthylformamide (178 ml) et de 78 ml d'eau sous agitation à 16 °C. On a ajouté à ce mélange dans l'ordre 4 g (5,7 mmole) de bis [5-amino-3-cyano-1-(2,6-dichloro-4-trifluorométhylphényl) pyrazol-4-yl] disulfure, 3,9 g de fluorotrichlorométhane, 3,96 g de dithionite de sodium. Le tout a été agité à 15-17°C pendant 1 heure puis versé sur de la glace puis le solide blanc a été filtré et lavé à l'eau (800 ml et séché. On a obtenu 4,34 g de produit attendu ( 84,1 % )
Point de fusion : 190-193°C

## Revendications

1. Procédé de préparation de perhalogenoalkylthioéthers caractérisé en ce que l'on met en contact, éventuellement dans un solvant
- un réducteur formé d'un métal choisi parmi le zinc, le cadmium, l'aluminium, le manganèse, avec du dioxyde de soufre, ou formé d'un dithionite alcalin ou d'un hydroxyméthane sulfinate alcalin, alcalino terreux ou métallique, ou formé d'un anion formiate et le dioxyde de soufre,
- un disulfure
- un halogénure de perfluoroalkyle.

2. Procédé selon la revendication 1, caractérisé en ce que l'halogénure de perfluoroalkyle répond à la formule :
XCFYT (II)
dans laquelle :
- Y et T représentent indépendamment l'un de l'autre un halogène choisi parmi le fluor,le chlore ou le brome ou une chaîne C₁- C₁₁ perhalogénoalkyle
- X représente un halogène choisi parmi le chlore, le brome et l'iode.

3. Procédé selon la revendication 2 caractérisé en ce que lorsque X représente le brome, Y et T représentent le fluor et lorsque X représente l'iode Y représente un chaîne perfluoroalkyle, T un atome de fluor.

4. Procédé selon la revendication 1 caractérisé en ce que le disulfure est choisi parmi les disulfures de formule:
R - S - S - R
dans laquelle :
- R représente un groupe hydrocarbyle, notamment un groupe carboacyclique (aliphatique) linéaire ou ramifié comportant une à cinq insaturations éthyléniques ou acétyléniques mais, de préférence, saturé, un groupe carbocyclique ou hétérocyclique choisi parmi un système monocyclique aromatique ou non aromatique (de préférence saturé), un système bicyclique aromatique ou non aromatique (de préférence saturé), un système polycyclique aromatique ou non aromatique (de préférence saturé), un système ponté (de préférence saturé).

5. Procédé selon la revendication 4, caractérisé en ce que R est un radical phényle éventuellement substitué par un ou plusieurs radicaux choisis parmi les radicaux suivants:
les atomes d'halogène (I,Cl,Br,F); les radicaux C₆- C₁₀ aryle éventuellement substitués par 1 à 6 substituants choisis parmi les atomes d'halogène (I,Cl,Br,F), les radicaux C₁- C₆ alkyle, C₁- C₆ alkoxy, polyhalo C₁- C₆ alkyle, polyhalo C₁- C₆ alkoxy; les radicaux C₁- C₉ hétéroaryle comportant en outre 1 à 4 hétéro atomes choisis parmi l'azote, le soufre, l'oxygène éventuellement substitué par l'un des substituants précédemment définis pour les radicaux C₆- C₁₀ aryle ; les radicaux C₁- C₆ alkoxy ; polyhalo C₁- C₆ alkoxy, les radicaux C₁- C₆ alkyle ou C₁- C₆ polyhaloalkyle ou R est un radical alkyle éventuellement substitué par un ou plusieurs radicaux choisis parmi les radicaux suivants:
les atomes d'halogène (I,Cl,Br,F); les radicaux C₆- C₁₀ aryle éventuellement substitués par 1 à 6
substituants choisis parmi les atomes d'halogène (I,Cl,Br,F), les radicaux C₁- C₆ alkyle, C₁- C₆ alkoxy, polyhalo C₁- C₆ alkyle, polyhalo C₁- C₆ alkoxy; les radicaux C₁- C₉ hétéroaryle comportant en outre 1 à 4 hétéro atomes choisis parmi l'azote, le soufre,
l'oxygène éventuellement substitué par l'un des substituants précédemment définis pour les radicaux C₆-C₁₀ aryle ; les radicaux C₁- C₆ alkoxy ; polyhalo C₁- C₆ alkoxy
où R répond à la formule : 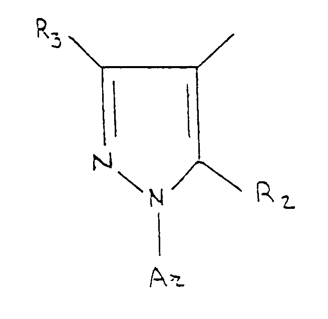 dans laquelle:
R₂ représente un groupe amino NR₄R₅ dans lequel R₄, R₅ identiques ou différents représentent un atome d'hydrogène ;un groupe C₁- C₆ alkyle éventuellement substitué par un groupe C₂- C₅ alkoxycarbonyle ; C₃- C₆ cycloalkyle ; C₂- C₇ alcanoyle éventuellement formant ensemble un groupe imide cyclique de 5 à 6 atomes avec l'atome d'azote auquel ils sont attachés, ledit groupe imide pouvant être éventuellement substitué par un à six atomes d'halogène ; C₂- C₇ alkoxycarbonyle ; polyhalo C₂-C₇ alkoxycarbonyle ; R₂ représente encore un groupe C₁-C₄ alkylsulfinyle ; C₂- C₅ alkoxyméthylène éventuellemnt substitué sur le méthylène par un groupe C₁- C₄ alkyle ; un atome d'halogène ; un groupe C₁- C₆ alkyle ; carboxy ; C₁- C₆ alkylthio ; polyhalo C₁- C₆ alkylthio ; C₁- C₆ alkylsufinyle ; polyhalo C₁- C₆ alkylsulfinyle, C₁- C₆ alkylsulfonyle, polyhalo C₁- C₆ alkylsulfonyle; tri C₁- C₆ alkylsilylmethyl ; tri C₁- C₆ alkylsilyl ; cyano ; hydroxy, hydroxy C₁- C₆ alkylamino, C₁- C₆ alkoxy
R₂ représente encore un reste HN- C(=A)- R₆, R₆ étant un atome d'hydrogène, un radical C₁- C₆ alkyle ; C₁- C₄ alkenyle ; C₁- C4 alkinyle ; C₁- C₄ alkoxyalkyle, C₁- C₄ alkylthioalkyle, C₁- C₄ alkoxy ; C₁- C₄ alkylthio, C₁- C₄ alkylamino, di(C₁- C₄ alkyl)amino ; polyhalo C₁-C₄ alkyle ; C₃- C₇ cycloalkyl éventuellement substitué par un ou plusieurs atomes d'halogène, C₁- C₄ alkyle ; C₁- C₄ halogènoalkyl ou R₆ représente encore un noyau phényl ; phénylthio ; phénoxy; phénylamino, ces noyaux phényl étant éventuellement substitués par les radicaux cyano, C₁- C₄ alkyl, C₁- C₄ alkoxy ; C₁- C₄ alkylthio ; C1- C₄ alkylsulfinyle ; C₁- C₄ alkylsulfonyle ; polyhalo C₁- C₄ alkyle ; polyhalo C₁- C₄ alkoxy ; polyhalo C₁- C₄ alkylthio ; polyhalo C₁- C₄ alkylsulfinyle ;polyhalo C₁-C₄ alkylsulfonyle ; les atomes d'halogène.
- A est NO₂, CO₂ C₁- C₆ alkyl, un atome de soufre ou d'oxygène,
- R₃ est un atome d'halogène ; un groupe cyano ou C₁- C₆ alkyle ; polyhalo C₁- C₆ alkyle ; C₃- C₆ cylcloalkyle
Ar est un noyau phényle ou pyridile éventuellement substitué par 1 à 4 substituants choisis parmi les radicaux cyano, C₁- C₄ alkyl, C₁- C₄ alkoxy ; C₁-C₄ alkylthio ; C₁- C₄ alkylsulfinyl ;C₁- C₄ alkylsulfonyl ; polyhalo C₁- C₄ alkyl ; polyhalo C₁- C₄ alkoxy ; polyhalo C₁- C₄ alkylthio ; polyhalo C₁- C₄ alkylsulfinyl ;polyhalo C1- C4 alkylsulfonyl ; les atomes d'halogènes

6. Procédé selon la revendication 5,caractérisé en ce que R répond à la formule 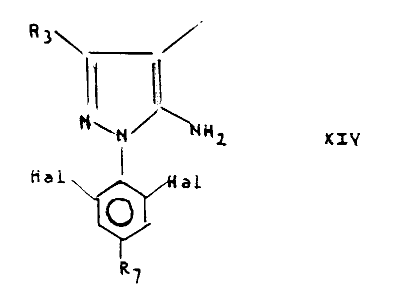 dans laquelle :
R₃ a la même signification que précédemment
Hal est un atome de fluor, de brome ou de chlore ou est un atome d'hydrogène
R₇ est un groupe trifluorométhyl ou trifluorométhoxy, R₇ est aussi un halogène.

7. Procédé selon la revendication 1 caractérisé en ce que la réaction est réalisée dans un solvant aprotique et suffisamment polaire choisi parmi, le formamide, le diméthylformamide, le diméthylacétamide, l'hexaméthylphosphoramide, la N- méthylpyrrolidone, le diméthylsulfoxyde, le sulfolane, les éthers comme le dioxanne, le tétrahydrofuranne, le diméthoxyéthane.

8. Procédé selon la revendication 1 caractérisé en ce que le métal utilisé est le zinc.

9. Procédé selon la revendication 1, caractérisé en ce que le dithionite alcalin, alcalino terreux ou métallique répond à la formule générale (XV) Mn(S₂O₄) dans laquelle n est égal à 1 ou 2 suivant la valence de métal M.

10. Procédé selon la revendication 1, caractérisé en ce que l'hydroxyméthane sulfinate est choisi parmi l'hydroxyméthane sulfinate de sodium ou de zinc

11. Procédé selon l'une des revendications 1, 9, 10, caractérisé en ce que lorsque l'on utilise un dithionite ou un hydroxyméthanesulfinate, on ajoute une base.

12. Procédé selon l'une des revendications 1, 8, 9, 10, 11, caractérisé en ce que la quantité molaire de zinc ou de dithionite ou d'hydroxyméthanesulfinate par rapport au disulfure est supérieure à 1.

13. Procédé selon la revendication 1, caractérisé en ce que les anions formiates proviennent de formiates de formule :
(HCOO-)ₙ R₁ⁿ⁺
R₁ⁿ⁺ étant égal à 1ou 2, étant choisi parmi les cations de métal alcalin (Na, K, Li), alcalino-terreux (Ca), ammonium de formule NR₂R₃R₄R₅, R₂, R₃, R₄, R₅, étant choisis parmi l'atome d'hydrogène, les radicaux C₃-C₁₈ alkyle, C₃-C₁₈ alcényle, C₂-C₁₈ alcynyle, lesdits radicaux étant éventuellement substitués par un radical hydroxy.

14. Procédé selon la revendication 13, caractérisé en ce que R₁ⁿ⁺ est choisi parmi les cations de métal alcalin, notamment le sodium, l'ammonium, l'isopropyl ammonium, le triéthylammonium, le triméthylammonium, le ter-butyl ammonium, l'éthanolammonium.

15. Procédé selon la revendication 1, 13, 14, caractérisé en ce que la quantité molaire de formiate par rapport au disulfure est supérieure à 1.

16. Procédé selon la revendication 1, caractérisé en ce que la quantité molaire d'halogénure de formule II mise en oeuvre par rapport au disulfure est supérieure à 1.

17. Procédé selon la revendication 1 ou 7, caractérisé en ce que lorsque l'halogénure de perfluoroalkyle est à l'état de gaz la réaction est effectuée en solvant qui dissout le gaz sous pressions de ce dernier, de préférence inférieure à 50 bars.

18. Procédé selon la revendication 1, caractérisé en ce que la température de réaction est comprise entre 20°C et 100°C ou à l'ébullition du solvant.

## Claims

1. Process for the preparation of perhaloalkylthioethers, characterized in that
- a reducing agent formed from a metal chosen from zinc, cadmium, aluminium and manganese, with sulphur dioxide, or formed from an alkali metal dithionite or of an alkali or alkaline-earth or metal hydroxymethane-sulphinate, or formed from a formate anion and sulphur dioxide,
- a disulphide, and
- a perfluoroalkyl halide
are brought into contact, optionally in a solvent.

2. Process according to Claim 1, characterized in that the perfluoroalkyl halide corresponds to the formula:
XCFYT (II)
in which:
- Y and T denote independently of each other a halogen chosen from fluorine, chlorine or bromine or a C₁-C₁₁ perhaloalkyl chain, and
- X denotes a halogen chosen from chlorine, bromine and iodine.

3. Process according to Claim 2, characterized in that when X denotes bromine, Y and T denote fluorine and when X denotes iodine Y denotes a perfluoroalkyl chain, and T a fluorine atom.

4. Process according to Claim 1, characterized in that the disulphide is chosen from disulphides of formula:
R - S - S - R
in which:
- R denotes a hydrocarbyl group, especially a linear or branched carboacyclic (aliphatic) group containing one to five ethylenic or acetylenic unsaturations but, preferably, saturated, a carbocyclic or heterocyclic group chosen from an aromatic or nonaromatic (preferably saturated) monocyclic system, an aromatic or nonaromatic (preferably saturated) bicyclic system, an aromatic or nonaromatic (preferably saturated) polycyclic system, or a bridged (preferably saturated) system.

5. Process according to Claim 4, characterized in that R is a phenyl radical optionally substituted by one or more radicals chosen from the following radicals:
halogen atoms (I, Cl, Br, F); C₆-C₁₀ aryl radicals optionally substituted by 1 to 6 substituents chosen from halogen atoms (I, Cl, Br, F), C₁-C₆ alkyl, C₁-C₆ alkoxy, polyhalo-C₁-C₆-alkyl and polyhalo-C₁-C₆-alkoxy radicals; C₁-C₉ heteroaryl radicals additionally containing 1 to 4 heteroatoms chosen from nitrogen, sulphur and oxygen, optionally substituted by one of the substituents defined above in the case of the C₆-C₁₀ aryl radicals; C₁-C₆ alkoxy radicals; polyhalo-C₁-C₆-alkoxy, C₁-C₆ alkyl or C₁-C₆ polyhaloalkyl radicals where R is an alkyl radical optionally substituted by one or more radicals chosen from the following radicals:
halogen atoms (I, Cl, Br, F); C₆-C₁₀ aryl radicals optionally substituted by 1 to 6 substituents chosen from halogen atoms (I, Cl, Br, F), C₁-C₆ alkyl, C₁-C₆ alkoxy, polyhalo-C₁-C₆-alkyl and polyhalo-C₁-C₆-alkoxy radicals; C₁-C₉ heteroaryl radicals additionally containing 1 to 4 heteroatoms chosen from nitrogen, sulphur and
oxygen optionally substituted by one of the substituents defined above in the case of the C₆-C₁₀ aryl radicals; C₁-C₆ alkoxy radicals; polyhalo-C₁-C₆-alkoxy
where R corresponds to the formula: 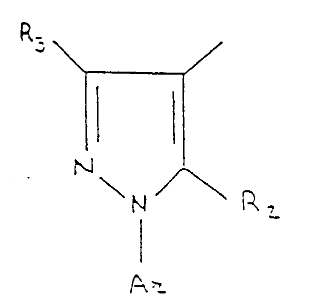 in which:
R₂ denotes an NR₄R₅ amino group in which R₄ and R₅, which are identical or different, denote a hydrogen atom; a C₁-C₆ alkyl group optionally substituted by a C₂-C₅ alkoxycarbonyl group; C₃-C₆ cycloalkyl; C₂-C₇ alkanoyl optionally forming a cyclic imide group of 5 to 6 atoms together with the nitrogen atom to which they are attached, it being possible for the said imide group to be optionally substituted by one to six halogen atoms; C₂-C₇ alkoxycarbonyl; polyhalo-C₂-C₇-alkoxycarbonyl; R₂ also denotes a C₁-C₄ alkylsulphinyl group; C₂-C₅ alkoxymethylene optionally substituted on the methylene by a C₁-C₄ alkyl group; a halogen atom; a C₁-C₆ alkyl group; carboxy; C₁-C₆ alkylthio; polyhalo-C₁-C₆-alkylthio; C₁-C₆ alkylsulphinyl; polyhalo-C₁-C₆-alkylsulphinyl, C₁-C₆ alkylsulphonyl, polyhalo-C₁-C₆-alkylsulphonyl; tri(C₁-C₆-alkyl)silylmethyl; tri(C₁-C₆-alkyl)silyl; cyano; hydroxyl, hydroxy-C₁-C₆ alkylamino, C₁-C₆ alkoxy
R₂ also denotes an HN-C(=A)-R₆ residue, R₆ being a hydrogen atom or a C₁-C₆ alkyl radical; C₁-C₄ alkenyl; C₁-C₄ alkynyl; C₁-C₄ alkoxyalkyl, C₁-C₄ alkylthioalkyl, C₁-C₄ alkoxy; C₁-C₄ alkylthio, C₁-C₄ alkylamino, di(C₁-C₄-alkyl)amino; polyhalo-C₁-C₄-alkyl; C₃-C₇ cycloalkyl optionally substituted by one or more halogen atoms or C₁-C₄ alkyl; C₁-C₄ haloalkyl or R₆ also denotes a phenyl nucleus; phenylthio; phenoxy; phenylamino, it being possible for these phenyl nuclei to be optionally substituted by cyano, C₁-C₄ alkyl or C₁-C₄ alkoxy radicals; C₁-C₄ alkylthio; C₁-C₄ alkylsulphinyl; C₁-C₄ alkylsulphonyl; polyhalo-C₁-C₄-alkyl; polyhalo-C₁-C₄-alkoxy; polyhalo-C₁-C₄-alkylthio; polyhalo-C₁-C₄-alkylsulphinyl; polyhalo-C₁-C₄-alkylsulphonyl; halogen atoms.
- A is NO₂, CO₂, C₁-C₆ alkyl, a sulphur or oxygen atom,
- R₃ is a halogen atom; a cyano or C₁-C₆ alkyl group; polyhalo-C₁-C₆-alkyl; C₃-C₆ cycloalkyl
Ar is a phenyl or pyridyl nucleus optionally substituted by 1 to 4 substituents chosen from cyano, C₁-C₄ alkyl and C₁-C₄ alkoxy radicals; C₁-C₄ alkylthio; C₁-C₄ alkylsulphinyl; C₁-C₄ alkylsulphonyl; polyhalo-C₁-C₄-alkyl; polyhalo-C₁-C₄-alkoxy; polyhalo-C₁-C₄-alkylthio; polyhalo-C₁-C₄-alkylsulphinyl; polyhalo-C₁-C₄-alkylsulphonyl; halogen atoms.

6. Process according to Claim 5, characterized in that R corresponds to the formula 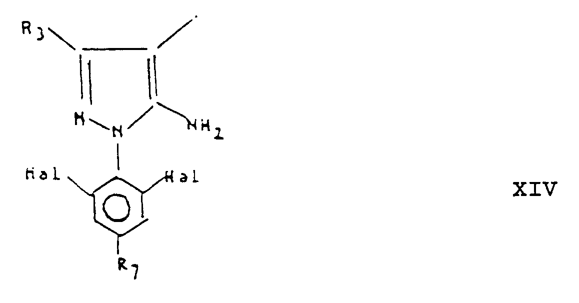 in which:
R₃ has the same meaning as above,
Hal is a fluorine, bromine or chlorine atom or is a hydrogen atom, and
R₇ is a trifluoromethyl or trifluoromethoxy group, R₇ is also a halogen.

7. Process according to Claim 1, characterized in that the reaction is carried out in an aprotic and sufficiently polar solvent chosen from formamide, dimethylformamide, dimethylacetamide, hexamethylphosphoramide, N-methylpyrrolidone, dimethyl sulphoxide, sulpholane, and ethers such as dioxane, tetrahydrofuran and dimethoxyethane.

8. Process according to Claim 1, characterized in that the metal employed is zinc.

9. Process according to Claim 1, characterized in that the alkali, alkaline-earth metal or metal dithionite corresponds to the general formula (XV) Mn(S₂O₄) in which n is equal to 1 or 2 depending on the valency of metal M.

10. Process according to Claim 1, characterized in that the hydroxymethanesulphinate is chosen from sodium or zinc hydroxymethanesulphinate.

11. Process according to one of Claims 1, 9 and 10, characterized in that when a dithionite or a hydroxymethanesulphinate is employed, a base is added.

12. Process according to one of Claims 1, 8, 9, 10 and 11, characterized in that the molar quantity of zinc or of dithionite or hydroxymethanesulphinate is greater than 1 relative to the disulphide.

13. Process according to Claim 1, characterized in that the formate anions originate from formates of formula:
(HCOO⁻)ₙR₁ⁿ⁺
R₁ⁿ⁺ being equal to 1 or 2, being chosen from cations of an alkali metal (Na, K, Li) or alkaline-earth metal (Ca) or ammonium of formula NR₂R₃R₄R₅, R₂, R₃, R₄ and R₅ being chosen from the hydrogen atom and C₃-C₁₈ alkyl, C₃-C₁₈ alkenyl and C₂-C₁₈ alkynyl radicals, the said radicals being optionally substituted by a hydroxyl radical.

14. Process according to Claim 13, characterized in that R₁ⁿ⁺ is chosen from the cations of an alkali metal, especially sodium, ammonium, isopropylammonium, triethylammonium, trimethylammonium, tert-butylammonium and ethanolammonium.

15. Process according to Claim 1, 13 and 14, characterized in that the molar quantity of formate is greater than 1 relative to the disulphide.

16. Process according to Claim 1, characterized in that the molar quantity of halide of formula II which is used is greater than 1 relative to the disulphide.

17. Process according to Claim 1 or 7, characterized in that when the perfluoroalkyl halide is in the gaseous state the reaction is carried out in a solvent which dissolves the gas under a pressure of the latter which is preferably lower than 50 bars.

18. Process according to Claim 1, characterized in that the reaction temperature is between 20°C and 100°C or at the boiling of the solvent.

## Patentansprüche

1. Verfahren zur Herstellung von Perhalogenalkylthioethern, dadurch gekennzeichnet, daß man gegebenenfalls in einem Lösungsmittel,
- ein Reduktionsmittel, gebildet aus einem Metall, ausgewählt aus Zink, Cadmium, Aluminium, Mangan mit Schwefeldioxid, oder gebildet aus einem Alkalidithionit oder einem Alkali-, Erdalkali- oder Metallhydroxymethansulfinat, oder gebildet aus einem Formiatanion und Schwefeldioxid,
- ein Disulfid und
- ein Perfluoralkylhalogenid,
zusammenbringt.

2. Verfahren nach Anspruch 1, dadurch gekennzeichnet, daß das Perfluoralkylhalogenid der Formel:
XCFYT (II)
entspricht, in der
- Y und T unabhängig voneinander ein Halogen, ausgewählt aus Fluor, Chlor und Brom oder eine C₁-C₁₁ Perhalogenalkylkette bedeuten, und
- X ein Halogen, ausgewählt aus Chlor, Brom und Iod, bedeutet.

3. Verfahren nach Anspruch 2, dadurch gekennzeichnet, daß, wenn X für Brom steht, Y und T jeweils Fluor bedeuten, und wenn X für Iod steht, Y eine Perfluoralkylkette und T ein Fluoratom bedeuten.

4. Verfahren nach Anspruch 1, dadurch gekennzeichnet, daß das Disulfid aus den Disulfiden der Formel:
R - S - S - R
ausgewählt wird, in der
- R eine Kohlenwasserstoffgruppe bedeutet, vorallem eine carboacyclische (aliphatische) lineare oder verzweigte Gruppe mit 1 bis 5 ethylenisch oder acetylenisch ungesättigten Bindungen, aber bevorzugt gesättigt, eine carbocyclische oder heterocyclische Gruppe, ausgewählt aus einem aromatischen oder nicht aromatischen (vorzugsweise gesättigten) monocyclischen System, einen aromatischen oder nicht aromatischen (vorzugsweise gesättigten) dicyclischen System, einem aromatischen oder nicht aromatischen (vorzugsweise gesättigten) polycyclischen System oder einem überbrückten (vorzugsweise gestättigten) System.

5. Verfahren nach Anspruch 4, dadurch gekennzeichnet, daß R eine Phenylgruppe ist, die gegebenenfalls substituiert ist durch eine oder mehrere Gruppen, ausgewählt aus den folgenden Gruppen:
Halogenatome (I, Cr, Br, F); C₆-C₁₀ Arylgruppen, gegebenenfalls substituiert durch 1 bis 6 Substituenten, ausgewählt aus Halogenatome (I, Cr, Br, F), C₁-C₆ Alkyl-, C₁-C₆ Alkoxy-, Polyhalogen-C₁-C₆-alkyl-, Polyhalogen-C₁-C₆-alkoxygruppen; C₁-C₉ Heteroarylgruppen, die außerdem 1 bis 4 Heteroatome enthalten, ausgewählt aus Stickstoff, Schwefel und Sauerstoff, gegebenenfalls substituiert durch einen der zuvor für die C₆-C₁₀ Arylgruppen definierten Substituenten; C₁-C₆ Alkoxygruppen; Polyhalogen-C₁-C₆-alkoxy-, C₁-C₆ Alkyl- oder C₁-C₆ Polyhalogenalkylgruppen,
oder daß R eine Alkylgruppe ist, die gegebenenfalls durch eine oder mehrere Gruppen substituiert ist, ausgewählt aus den folgenden Gruppen:
Halogenatome (I, Cl, Br, F); C₆-C₁₀ Arylgruppen, gegebenenfalls substituiert durch 1 bis 6 Substituenten, ausgewählt aus Halogenatomen (I, Cl, Br, F), C₁-C₆ Alkyl-, C₁-C₆ Alkoxy-, Polyhalogen-C₁-C₆-alkyl- und Polyhalogen-C₁-C₆-alkoxygruppen; C₁-C₉ Heteroarylgruppen, die außerdem 1 bis 4 Heteroatome, ausgewählt aus Stickstoff, Schwefel und Sauerstoff, enthalten, gegebenenfalls substituiert durch einen der zuvor für die C₆-C₁₀ Arylgruppen definierten Substituenten; C₁-C₆ Alkoxygruppen; Polyhalogen-C₁-C₆-alkoxygruppen;
oder R entspricht der Formel: 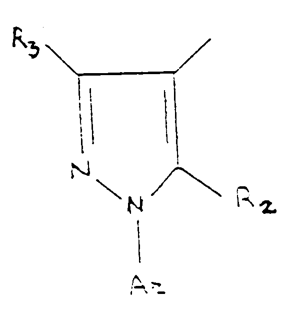 darin bedeutet R₂ eine Aminogruppe NHR₄R₅, bei der R₄ und R₅ gleich oder verschieden sind und eine der folgenden Bedeutungen haben: Wasserstoffatom; eine C₁-C₆ Alkylgruppe, die gegebenenfalls durch eine C₂-C₅ Alkoxycarbonylgruppe substituiert ist; C₃-C₆ Cycloalkyl; C₂-C₇ Alkanoyl, das gegebenenfalls zusammen mit dem Stickstoffatom eine cyclische Imidgruppe mit 5 bis 6 Atomen bildet, wobei die Imidgruppe gegebenenfalls durch 1 bis 6 Halogenatome; C₂-C₇ Alkoxycarbonyl; Polyhalogen-C₂-C₇-alkoxycarbonyl; substituiert sein kann; R₂ bedeutet weiterhin eine C₁-C₄ Alkylsulfenylaminogruppe; eine C₂-C₅ Alkoxymethylengruppe, gegebenenfalls substituiert am Methylen durch eine C₁-C₄ Alkylgruppe; ein Halogenatom; eine C₁-C₆ Alkyl-gruppe; Carboxy; C₁-C₆ Alkylthio; Polyhalogen-C₁-C₆-alkyl-thio; C₁-C₆ Alkylsulfinyl; Polyhalogen-C₁-C₆-alkylsulfinyl, C₁-C₆ Alkylsulfonyl, Polyhalogen-C₁-C₆-alkylsulfonyl; Tri-C₁-C₆-alkylsilylmethyl; Tri-C₁-C₆-alkylsilyl; Cyano; Hydroxy; Hydroxy-C₁-C₆-alkylamino, C₁-C₆ Alkoxy.
R₂ bedeutet noch einen Rest HN-C(=A)-R₆, wobei R₆ ein Wasserstoffatom eine C₁-C₆ Alkylgruppe; C₂-C₄ Alkenyl; C₂-C₄ Alkinyl; C₁-C₄ Alkoxyalkyl, C₁-C₄ Alkyl-thioalkyl, C₁-C₄ Alkoxy; C₁-C₄ Alkylthio, C₁-C₄ Alkylamino, Di(C₁-C₄ alkyl)amino; Polyhalogen-C₁-C₄-alkyl; C₃-C₇ Cycloalkyl, gegebenenfalls substituiert durch ein oder mehrere Halogenatome, C₁-C₄ Alkyl; C₁-C₄ Halogenalkyl; oder R₆ bedeutet auch eine Phenylgruppe; Phenylthio; Phenoxy; oder Phenylamino, wobei diese Phenylgruppen gegebenenfalls durch folgende Gruppen substituiert sein können, C₁-C₄ Alkyl, C₁-C₄ Alkoxy; C₁-C₄ Alkylthio; C₁-C₄ Alkylsulfinyl; C₁-C₄ Alkylsulfonyl; Polyhalogen-C₁-C₄-alkyl; Polyhalogen-C₁-C₄-alkoxy; Polyhalogen-C₁-C₄-alkylthio; Polyhalogen-C₁-C₄-alkylsulfinyl; Polyhalogen-C₁-C₄-alkylsulfonyl; Halogenatome.
- A ist NO₂, CO₂ C₁-C₆ Alkyl, ein Schwefel- oder Sauerstoffatom,
- R₃ ist ein Halogenatom, eine Cyano- oder C₁-C₆ Alkylgruppe; Polyhalogen-C₁-C₆-alkylgruppe oder C₃-C₆ Cycloalkylgruppe;
Ar ist eine Phenyl- oder Pyridylgruppe, gegebenenfalls substituiert durch 1 bis 4 Substituenten, ausgewählt unter folgenden Gruppen: Cyan, C₁-C₄ Alkyl; C₁-C₄ Alkoxy; C₁-C₄ Alkylthio; C₁-C₄ Alkylsulfinyl; C₁-C₄ Alkylsulfonyl; Polyhalogen-C₁-C₄-alkyl; Polyhalogen-C₁-C₄-alkylthio; Polyhalogen-C₁-C₄-alkylsulfinyl; Polyhalogen-C₁-C₄-alkylsulfonyl; Halogenatome.

6. Verfahren nach Anspruch 5, dadurch gekennzeichnet, daß R der Formel: 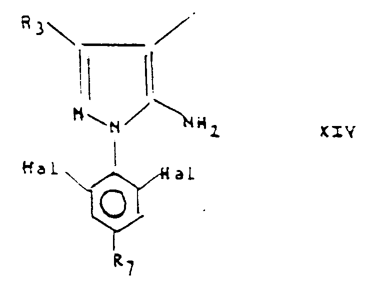 entspricht, in der R₃ die gleiche Bedeutung wie zuvor hat, Hal ein Fluor-, Brom- oder Chloratom oder ein Wasserstoff ist, und
R₇ für eine Trifluormethyl- oder Trifluormethoxygruppe steht, und R₇ auch ein Halogen sein kann.

7. Verfahren nach Anspruch 1, dadurch gekennzeichnet, daß die Reaktion in einem aprotischen und ausreichend polaren Lösungsmittel durchgeführt wird, das aus Formamid, Dimethylformamid, Dimethylacetamid, Hexamethylphosphoramid, N-Methylpyrrolidon, Dimethylsulfoxid, Sulfolan und Ethern wie Dioxan, Tetrahydrofuran, Dimethoxyethan ausgewählt wird.

8. Verfahren nach Anspruch 1, dadurch gekennzeichnet, daß das verwendete Metall Zink ist.

9. Verfahren nach Anspruch 1, dadurch gekennzeichnet, daß das Alkali-, Erdalkali- oder Metalldithionit der allgemeinen Formel (XV) Mₙ(S₂O₄) entspricht, in der n gleich 1 oder 2 ist, je nach der Wertigkeit des Metalls M.

10. Verfahren nach Anspruch 1, dadurch gekennzeichnet, daß als Hydroxymethansulfinat Natrium- oder Zinkhydroxymethansulfinat gewählt wird.

11. Verfahren nach einem der Ansprüche 1, 9 und/oder 10, dadurch gekennzeichnet, daß bei Verwendung eines Dithionits oder eines Hydroxymethansulfinats eine Base zugegeben wird.

12. Verfahren nach einem der Ansprüche 1, 8, 9, 10 und/oder 11, dadurch gekennzeichnet, daß die Molmenge Zink, des Dithionits oder des Hydroxymethansulfinats,bezogen auf Disulfid, mehr als 1 beträgt.

13. Verfahren nach Anspruch 1, dadurch gekennzeichnet, daß die Formiatanionen von Formiaten der Formel
(HCOO-)ₙ R₁ⁿ⁺
geliefert werden, wobei R₁ⁿ⁺, mit n gleich 1 oder 2, unter den Alkalimetallkationen (Na, K, Li), Erdalkalikationen (Ca) und Ammonium der Formel NR₂R₃R₄R₅ ausgewählt wird und R₂, R₃, R₄, R₅ ausgewählt werden aus Wasserstoffatom und den Gruppen C₁-C₁₈ Alkyl, C₂-C₁₈ Alkenyl, C₂-C₁₈ Alkinyl, wobei diese Gruppen gegebenenfalls durch eine Hydroxygruppe substituiert sein können.

14. Verfahren nach Anspruch 13, dadurch gekennzeichnet, daß R₁ⁿ⁺ ausgewählt wird aus Alkalimetallkationen, vorallem Natrium, aus Ammonium, Isopropylammonium, Triethylammonium, Trimethylammonium, ter-Butylammonium und Ethanolammonium.

15. Verfahren nach Anspruch 1, 13 und/oder 14, dadurch gekennzeichnet, daß die Molmenge Formiat, bezogen auf Disulfid, mehr als 1 beträgt.

16. Verfahren nach Anspruch 1, dadurch gekennzeichnet, daß die Molmenge des eingesetzten Halogenids der Formel II, bezogen auf Disulfid, mehr als 1 beträgt.

17. Verfahren nach Anspruch 1 oder 7, dadurch gekennzeichnet, daß, wenn das Perfluoralkylhalogenid gasförmig vorliegt, die Reaktion in einem Lösungsmittel ausgeführt wird, welches das Gas unter Drücken des letzteren von vorzugsweise weniger als 50 bar löst.

18. Verfahren nach Anspruch 1, dadurch gekennzeichnet, daß die Reaktionstemperatur im Bereich von 20 bis 100°C oder dem Siedepunkt des Lösungsmittels liegt.
